(19) Europäisches Patentamt
European Patent Office
Office européen des brevets

(11) **EP 2 066 286 B1**

(12) **EUROPEAN PATENT SPECIFICATION**

(45) Date of publication and mention
of the grant of the patent:
**07.12.2011 Bulletin 2011/49**

(51) Int Cl.:
*A61K 8/04* *(2006.01)*        *A61K 8/34* *(2006.01)*
*A61Q 17/00* *(2006.01)*      *A61Q 17/04* *(2006.01)*
*A61K 8/73* *(2006.01)*

(21) Application number: **07801396.8**

(22) Date of filing: **20.09.2007**

(86) International application number:
**PCT/DK2007/050131**

(87) International publication number:
**WO 2008/034447 (27.03.2008 Gazette 2008/13)**

(54) **EMULSION**

EMULSION

ÉMULSION

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**HR**

(30) Priority: **20.09.2006  DK 200601213
20.09.2006  US 845753 P**

(43) Date of publication of application:
**10.06.2009  Bulletin 2009/24**

(60) Divisional application:
**11179538.1**

(73) Proprietor: **Riemann Trading ApS
3400 Hillerød (DK)**

(72) Inventor: **FAIYAZIANNASAB, Flora
DK-2200 Copenhagen N (DK)**

(74) Representative: **Dalkiær, Mikkel et al
Plougmann & Vingtoft A/S
Sundkrogsgade 9
P.O. Box 831
DK-2100 Copenhagen Ø (DK)**

(56) References cited:
**EP-A- 0 861 657        WO-A-98/10742
WO-A-2007/140442  US-A- 4 686 099
US-A- 5 017 366        US-A1- 2002 172 646**

Remarks:
The file contains technical information submitted after
the application was filed and not included in this
specification

**Description**

**Field of the invention**

The present invention relates to a stabile emulsion-type product. In particular the present invention relates to a stabile emulsion-type product comprising a mono-alcohol, water, hydrophilic colloid and a water immiscible component, which is useful as a carrier, in particular a carrier of sun screen filter(s). Background of the invention :

It is a well established fact that light radiation of wavelengths which are within the UV range of from 280 nm to 400 nm is desirable in that it promotes tanning of human skin. However UV irradiation also causes skin bums, loss of skin elasticity and the appearance of wrinkles, promoting premature skin aging. In addition, UV irradiation promotes the triggering of the erythemal reaction or accentuates this reaction in certain individuals and may even be the source of phototoxic or photoallergic reactions. Last, but not least important, UV irradiation has carcinogenic effects. For these reasons, continuous need exists for means for controlling natural tanning/bronzing and, thus, for controlling the coloration of the skin.

Numerous requirements exist for dermatological compositions for photo-protection of the human skin: Compositions are preferred which have a pleasant fee and are formulated as non-greasy milks and creams that are easily spread. In particular, products for use in the sun are preferably products that will be perceived as light, clear and, if possible, also with a cooling effect. it is further preferred that the compositions exhibit non-adherence to sand, as children are often in contact with sand at beaches and play areas.

In addition to being convenient for the user the compositions must contain adequate solvent to hold the necessary amount of UV-filter need continues to exist for compositions which are simultaneously free of preservatives, while exhibiting an optimum stability which is maintained throughout the time of storage and ultimate use of the products. Finally, in instances where a particularly high and lasting protection is desired, as for children's skin or for sensitive skin, it is also preferable to employ compositions which exhibit good water persistence and which thus retain a very good photo protective power even after bathing several times.

In particular for sensitive skin types, there is also a desire to minimize the risk of sensitization while avoiding, as far as possible, the presence within the formulations of active agents capable of causing skin reactions. Thus, need continues to exist for compositions which are simultaneously free of preservatives, while exhibiting an optimum stability which is maintained throughout the time of storage and ultimate use of the products.

Many photoprotective, dermatological compositions are oil-in-water emulsions. However, one of the disadvantages of traditional oil-in-water emulsions is that they very easily lose their effectiveness with respect to UV protection as soon as they come into contact with water; this because the screening agents which they contain in their aqueous phase are removed by the water, during bathing in the sea or in swimming pools, for example, or, alternatively, under the shower or when playing water sports, and the overall photoprotective power of these compositions thus is greatly reduced.

Micro-emulsions have been prepared, in which the dispersed phase is essentially solubilized in the continuous phase by the action of a surfactant. Ordinarily, however, these systems require fairly large quantities of surfactant whereas, normally, it is desirable to keep surfactant levels low in cosmetic compositions because they can reduce the natural lipid barrier of the skin, and thus leave the skin more susceptible to irritants.

Currently used vehicles for photoprotective agents are nano-emulsions containing small sized oil droplets formed by processing the components under high pressure. While providing a vehicle that is elegant in its appearance, the nano-emulsion is a system in very delicate balance, subject to disruption if the wrong component is added. In addition, the process of manufacturing a nano-emulsion is relatively costly and the known nano-emulsion systems may rely on the presence of relatively high levels of emulsifiers that are considered irritating, such as ethoxylated fatty ethers and esters.

Hence, an improved product to be used as a vehicle for sunscreen agents and other cosmetic agents would be one that combines ease of manufacture, stability, safety and convenience for the end user as evaluated on the basis of the above considerations.

**Summary of the invention**

Thus, an object of the present invention relates to a stable emulsion-type product comprising at least 10%(w/w) of a mono alcohol having 2-8 carbon atoms, 1-6%(w/w) water, a hydrophilic colloid and at least one water immiscible component.

In particular, it may be seen as an object of the present invention to provide a stable emulsion-type product, wherein the product is a sunscreen product.

It is a further object of the present invention to provide a stable emulsion-type product, wherein the product is a cosmetic.

Yet another aspect of the present invention is to provide a method for producing a stable emulsion-type product comprising at least 10%(w/w) of a mono alcohol having 2-8 carbon atoms, 1-6%(w/w) water, a hydrophilic colloid and at least one water immiscible component, said method comprises the steps of:

(i) Providing a water immiscible phase comprising one or more water immiscible components

(ii) mixing the at least 10%(w/w) mono alcohol having 2-8 carbon atoms with the 1-6%(w/w) and the hydrophilic colloid water providing a water/alcohol-mixture, and

(iii) mixing the water immiscible phase from step (i) with the water/alcohol-mixture in step (ii) and obtaining the stable emulsion-type product.

Still another aspect of the present invention pertains to a formulation comprising a stable emulsion-type product comprising at least 10%(w/w) of a mono alcohol having 2-8 carbon atoms, 1-6% (w/w) water, a hydrophilic colloid and at least one water immiscible component, and an active ingredient.

A further aspect of the invention includes:

The non-therapeutic use of an emulsion-type product according to the present invention, as a carrier substance for an active ingredient.

**Definitions**

Prior to discussing the present invention in further details, the following terms and conventions will first be defined:

In the present context the term "emulsion-type product" refers to the mixing of two or more types of liquid which as such are not intermixable to provide a stabile solution of said non-intermixable liquids. In an embodiment of the present invention the stabile solution of the two or more types of liquid which as such are not intermixable, may be provided without the use of nano-emulsion techniques or the likes.

In the present context the term "emollient" refers to a substance added to a formulation that gives it softening ability. For example, oils that can soften skin are added as emollients in some skin creams.

In the present context the term "at most" e.g. in connection to the content of water in the product, relates to the highest concentration of water as well as the presence of water. Thus, in the present context the term "at most" does not mean that water content may be 0%.

In the present context the term "alcohol" refers to an alcohol having 2-8 carbon atoms. Preferably, the alcohol comprised herein may be an alcohol having an un-branched carbon chain.

Furthermore, when used in relation to the present invention the term "alcohol" refers to an organic compound in which a hydroxyl group (-OH) is attached to a saturated carbon atom. Alcohols have the general formula ROH, where R may be a lower-aliphatic or cyclic and may include aromatic rings. Alcohols are further classified as primary, secondary or tertiary, according to the position of the hydroxyl group on the lower-aliphatic carbon chain..

Alcohols may also be classified by the number of hydroxyl groups present in the molecule; for example, a compound with two hydroxyl groups (a diol), and with three hydroxyl groups (a triol).

In the present context the term "Alcohol Denaturant" relates to ethyl alcohol that is denatured with one or more denaturing agents. One such denaturing agents may be Denatonium Benzoate.

Alcohols may be widely used in cosmetics. The low molecular weight alcohols, e.g., isopropyl, butyl, and especially ethyl alcohol, are employed as solvents as well as for their astringent action in e.g. aftershaves, colognes, mouthwashes, and similar products.

In the present context the term "water immiscible phase" refers to a chemical compound or a mixture of chemical compounds that is substantially non-miscible with water, and which is in a liquid state at ambient temperatures. In the context of the present invention the term comprisesalkoxylated alcohols, aliphatic alcohols, siloxanes; silanes; hydrophilic colloids; synthetic polymers and oils including esters, as well as combinations of these.

The term "water immiscible component" refers to a member of a "water immiscible phase" as defined above. In particular, the water immiscible component may be an oil, such as an ester or a siloxanes or a silanes or a combination hereof.

In the present context the term "higher-aliphatic alcohol" relates to non-aromatic organic compounds, in which carbon atoms are joined together in straight or branched chains rather than in benzene rings. The higher-aliphatic alcohols include not only saturated alkanes, but also unsaturated compounds, such as alkenes or alkynes.

In the context of the present invention, the term "alkoxylated alcohol" refers to an organic compound, such as an alkyl-group, comprising an alcohol group and linked to an oxygen atom. Preferably, the alkoxylated alcohol is a PPG-15 Stearyl ether, PPG15 or a combination thereof.

The term "hydrophilic colloid" refers to a colloidal dispersion in which the dispersed particles are more or less liquid and exert a certain attraction on and absorb a certain quantity of the fluid in which they are suspended.

The term "synthetic polymer" refers to organic molecules consisting of structural units and a large number of repeating units connected by covalent chemical bonds which are put together by design- or by natural processes. In a preferred embodiment of the present invention the synthetic polymer is a copolymer. In the present context the term "copolymer" relates to a polymer formed when two (or more) different types of monomer are linked in the same polymer chain, as opposed to a homopolymer where only one monomer is used. In an embodiment of the present invention the two different types of monomers used in the copolymer of the present invention are an acrylate and an octylacrylamide.

In the present context the term "minimal erythema dose (MED)" refers to the quantity of erythema-effective energy (expressed as Joules per square meter) required to produce the first perceptible, redness reaction in the skin with clearly defined borders.

"Product category designation" (PCD) is a labelling designation for sunscreen drug products to aid in selecting the type of product best suited to an individual's complexion (pigmentation) and desired response to ultraviolet (UV) radiation. The designation is according to recommendations by the US Food and Drug Administration.

> (1) *Minimal sun protection product*. A sunscreen product that provides a sun protection factor (SPF) value of 2 to under 12.

> (2) *Moderate sun protection product*. A sunscreen product that provides an SPF value of 12 to under 30.

> (3) *High sun protection product*. A sunscreen product that provides an SPF value of 30 or above.

Slightly different guidelines for labelling of SPF number and product categories are suggested by COLIPA (The European Cosmetic Toiletry and perfumery Association) in Europe as illustrated in Example 4 of the present application.

In the present context the term "Sun protection factor (SPF) value" refers to the UV energy required to produce an MED on protected skin divided by the UV energy required to produce an MED on unprotected skin. The "sun protection factor" term may also be defined as the ratio of the minimum erythemal dose on protected skin ($MED_p$) to the minimum erythemal dose on unprotected skin ($MED_u$):

$$SPF = MED_p/MED_u$$

The Sun Protection Factor value on an individual subject (SPF1), for any product, either before or after water immersion, may be determined as the ratio of the minimum erythemal dose on protected skin ($MED_p$) to the minimum erythemal dose on unprotected skin ($MED_u$) of the same subject.

Further, the term "static sun protection factor", (SPFs), relates to the sun protection factor before water immersion, while the term "wet sun protection factor" ($SPF_w$) refers to the sun protection factor after water immersion.

The static and wet SPFs are determined according to the current published International Sun Protection Factor (SPF) Test Method (COLIPA document number 001-2003; February 2003).

The term "active ingredient" refers to one or more components present in the emulsion type product capable of execute a desired function, e.g. when applied to the skin of a human. In a preferred embodiment of the present invention the active ingredient is a sun protection filter.

The term "sunscreen product" refers to a formulation comprising one or more sunscreen agents. The term "sunscreen agent" relates to an active ingredient that absorbs or reflects radiation in the UV range at wavelengths from 290 to 400 nanometers.

The term "cosmetic product" refers to a substances intended to be applied to the human body for cleansing, beautifying, promoting attractiveness, or altering the appearance without affecting the body's structure or functions. Included in this definition are skin creams, lotions, perfumes, lipsticks, fingernail polishes, eye and facial makeup preparations, permanent waves, hair colours, toothpastes, and deodorants, as well as any material intended for use as a component of a cosmetic product.

## DETAILED DESCRIPTION OF THE INVENTION

Emulsion-type product

In a first aspect the present invention provides a stable emulsion-type product comprising at least 10% (w/w) of a mono alcohol having 2-8 carbon atoms, 1-6% (w/w) water and at water immiscible phase. As demonstrated in the present application the inventors have surprisingly found that the mono alcohol, water, a hydrophilic colloid and water immiscible components combined in the specified relative amounts form an emulsion-type product which is stable and does not form any precipitate. The product is easily applied to a surface such as human or animal skin and is convenient when used e.g. as a vehicle for agents that are to be applied to the surface.

In particular embodiments the mono alcohol of the emulsion-type product has 2-7 carbon atoms, such as 2-6, 2-5, 2-4 or 2-3 carbon atoms. Specifically, it may be preferred that the mono alcohol has 2, 3, 4, 5, 6, 7 or 8 carbon atoms. In a specific embodiment according to the invention the mono alcohol is an alcohol having 2-4 carbon atoms. It will be understood that the specific choice of mono alcohol may depend on the particular purpose for which the emulsion-type product is contemplated.

According to a further specific embodiment the alcohol is an alcohol having an un-branched carbon chain.

In preferred embodiments the alcohol is selected from the group consisting of ethanol, 1-propanol, 2-propanol, ethylene glycol, 1-butanol, 2-butanol or any combination thereof.

It may further be preferred that the alcohol content of the emulsion type product of the invention is in the range of 10-90%(w/w), such as in the range of 15-90%(w/w), e.g. in the range of 20-85%(w/w), such as in the range of 30-80% (w/w), e.g. in the range of 40-75%(w/w), such as in the range of 50-70%(w/w), e.g. in the range of 55-65%(w/w), such as about 60 %(w/w).

In equally preferred embodiments of the invention the water content of said emulsion type product is 1-6% (w/w), e.g. in the range of 1-5%(w/w), 2-6%(w/w), 2-5%(w/w), 3-6%(w/w), 3-5%(w/w), 4-6%(w/w), or 5%(w/w).

Additionally, preferred emulsion-type products of the invention may comprise a water content of less than 6% (w/w), such as less than 5%(w/w), such as less than 4%(w/w), such as less than 3%(w/w), such as less than 2%(w/w).

According to additionally preferred embodiments of the invention the emulsion-type product according to the invention has a conductivity less than 4 ms/cm, such as a conductivity of 3 ms/cm or less, e.g. a conductivity of 2 ms/cm or less, such as a conductivity of 1 ms/cm or less, e.g. a conductivity of 500 $\mu$s/cm or less, such as a conductivity of 250 $\mu$s/cm or less, e.g. a conductivity of 100 $\mu$s/cm or less, such as a conductivity of 50 $\mu$s/cm or less, when measuring on a mixture of 50%(w/w) product and 50%(w/w) water at 20°C.

In further embodiments the emulsion-type product of the invention comprises more than one water immiscible components, such as at least two water immiscible components, e.g. at least 3 water immiscible components, 4 water immiscible components, such at least 5 water immiscible components. In certain embodiments of the invention at least one of the water immiscible components present in the product is characterised in that it is not miscible with at least one other water immiscible component present in the product. In other words, the product comprises, according to some embodiments of the invention, at least two water immiscible components that are not intermixable or mutually soluble.

In similarly preferred embodiments of the invention the emulsion-type product has a pH of 4 or above, e.g. a pH of 5 or above, such as pH 6 or above, e.g. pH 7 or above, such as pH 8 or above, when measuring on a mixture of 50% (w/w) product and 50% (w/w) water at 20°C.

Accordingly, it may be preferred that the product has a pH in the range of 4-10, such as in the range of 5-10, such as in the range of 6-10, such as in the range of 5-9, such as in the range of 5-8, such as in the range of 5-7, such as in the range of 6-9, such as in the range of 6-8, such as in the range of 6-7, e.g. in the range of 7-9 or in the range of 7-8, when measuring on a mixture of 50% (w/w) product and 50% (w/w) water at 20°C.

For specific purposes it may be desired to obtain a lower or higher pH and the addition of acid or alkali components to adjust the pH may be contemplated.

The emulsion-type product according to the invention may further comprise an alkoxylated alcohol. Suitable examples of alkoxylated alcohols are PPG-15 Stearyl ether, PPG-15 and PPG-11 Stearyl ether. In preferred embodiments of the invention the alkoxylated alcohol is PPG-15 Stearyl ether or PPG-15 or a combination thereof.

As for the amount of alkoxylated alcohols present in the emulsion-type product according to the invention it may be preferred that the alkoxylated alcohol is added in an amount in the range of 0.1-20%(w/w), such as in the range of 0.5-15%(w/w), e.g. in the range of 1-10%(w/w), such as in the range of 2-7%(w/w), e.g. about 5%(w/w).

In further preferred embodiments according to the invention the emulsion-type product further comprises a higher-aliphatic alcohol having 10 carbon atoms or more. Examples of suitable higher-aliphatic alcohols are hexyldecanol and hexyldecyloctadecanol.

According to preferred embodiments of the invention the higher-aliphatic alcohol having 10 carbon atoms or more is added in an amount In the range of 0.1-20%(w/w), such as in the range of 0.5-15%(w/w), e.g. in the range of 1-10%(w/w), such as in the range of 2-7%(w/w), e.g. about 6%(w/w).

In additional preferred embodiments of the invention the emulsion-type product further comprises a siloxane or a silane. Suitable examples of silanes and siloxanes are cyclomethicone, cyclohexasiloxane cycloheptasiloxane cyclotrisiloxane and cydotetrasiloxane. In currently most preferred embodiments of the invention, the siloxane is cyclic dimethyl polysiloxane or cyclopentasiloxane.

According to the invention it is preferred that the the siloxane or the silane is added in an amount in the range of 0.1-20%(w/w), such as in the range of 0.5-15%(w/w), e.g. in the range of 1-10%(w/w), such as in the range of 2-7% (w/w), e.g. in the range of 2.5-5%(w/w). Preferably, the siloxane or the silane is added in an amount above 2.5 %(w/w), e.g. above 3 %(w/w), such as above 4 %(w/w), e.g. above 5 %(w/w), such as above 7 %(w/w), e.g. above 8 %(w/w). Alternatively, the siloxane or the silane is added in an amount below 20 %(w/w), e.g. below 15 %(w/w), such as below 10 %(w/w).

According to the currently most preferred embodiments of the invention the emulsion-type product of the invention comprises a combination of an alkoxylated alcohol, a higher-aliphatic alcohol having 10 carbon atoms or more and/or a siloxane or a silane.

In further embodiments of the invention the emulsion-type product further comprises a hydrophilic colloid (hydrophilic colloid and derivatives or gums). In this context suitable hydrophilic colloids may be selected from the group consisting of: hydroxypropyl ether and Hydroxypropylcellulose or combinations thereof. In particularly preferred embodiments the hydrophilic colloid is Hydroxypropylcellulose.

According to the invention, the hydrophilic colloid is preferably added in an amount in the range of 0.01-5% (w/w), such as in the range of 0.1-2%(w/w), e.g. in the range of 0.2-1%(w/w), such as in the range of 0.2-0.7%(w/w), e.g. about 0.4%(w/w).

In still further embodiments of the invention the emulsion-type product comprises a synthetic polymer. Suitable synthetic polymers may be selected from the group consisting of: acrylate, octylacrylamide and one or more monomers consisting of acrylic acid, methacrylic acid and one of their esters, or combinations thereof. In even more preferred embodiments the synthetic polymer is an acrylate and/or an octylacrylamide copolymer.

According to the invention it is preferred that the synthetic polymer is added in an amount in the range of 0.1-20%(w/w), such as in the range of 0.5-15%(w/w), e.g. in the range of 1-10%(w/w), such as in the range of 1.5-5% (w/w), e.g. about 2%(w/w).

An emulsion-type product according to any one of the preceding claims, wherein the product further comprises an ester (or amines or phenols). In currently most preferred embodiments of the invention the ester is selected from the group consisting of: ethylhexyl methoxycinamate, diethylamino hydroxybenzoyl hexyl benzoate, and octocrylene and any combination thereof.

In further preferred embodiments of the invention the ester is added to said emulsion-type product in an amount in the range of 0.1-20%(w/w), such as in the range of 0.5-15%(w/w), e.g. in the range of 1-10%(w/w), such as in the range of 2-7%(w/w), e.g. about 5%(w/w).

In a preferred embodiment of the present invention the emulsion-type product comprises a mono alcohol having 2-8 carbon atoms, water, a hydrophilic colloid, at least one water immiscible component and at least one component selected from a siloxane or a silane, higher-aliphatic alcohol having 10 carbon atoms or more and an alkoxylated alcohol, such as at least two of these components e.g. three of these components. Preferably, the emulsion-type product comprises a mono alcohol having 2-8 carbon atoms, water, a hydrophilic colloid, at least one water immiscible component and a siloxane or a silane. Siloxane and silane may also be considered being a water immiscible component. Siloxane and silane may be miscible in the lower alcohols and several organic solvents, preferably, siloxane and silane may be completely miscible in the lower alcohols and several organic solvents. The concentrations of the components are similar to the concentrations mentioned earlier in the description.

The emulsion-type product according to the invention is preferably a sunscreen product or a cosmetic product. According to one particular embodiment the sunscreen product is a sunscreen lip protectant. According to other particular embodiments, the cosmetic product is selected from the group consisting of lip-glosses, a foundation, lipsticks and lip moisturizers.

According to further embodiments the cosmetic product of the invention is a hair care product.

In the currently most preferred embodiment of the invention the emulsion-type product is a sunscreen product. When developed as a sunscreen product, the emulsion-type product according to the present invention provides a number of important advantages including the fact that it is easy to apply, non-greasy and has a cooling effect.

When used as a sunscreen product, the emulsion-type product of the invention may comprise any currently known compound capable of functioning as a sunscreen agent as defined hereinbefore. A range of sunscreen agents approved by the U.S. Food and Drug Administration are listed in Code of Federal Regulations title 21 (21 CFR 352.10). These include: Aminobenzoic acid (PABA), Avobenzone, Cinoxate, Dioxybenzone, Homosalate, Menthyl anthranilate, , Octocrylene, Octyl methoxycinnamate, Octyl salicylate, Oxybenzone, Padimate O, Phenylbenzimidazole sulfonic acid, Sulisobenzone, Titanium dioxide, Trolamine salicylate, Zinc oxide, Benzophenone-3, Ethylhexyl Methoxycinnamate, Octocrylene, 4-tert-Butyl-4'-methoxy-dibenzoylmethane (BMBM) or Diethylamino Hydroxybenzoyl Hexyl Benzoate.

Preferably Benzophenone-3 is used in concentrations of at the most 10 %(w/w), e.g. in the range of 0.1-10 % (w/w), such as in the range of 0.5-8 %(w/w), e.g. in the range 1-6 %(w/w), such as in the range of 3-5 %(w/w), e.g. in the range 6-10 %(w/w), such as in the range of 8-10 %(w/w) and/or Diethylamino Hydroxybenzoyl Hexyl Benzoate is used in concentrations of at the most 10 %(w/w), e.g. in the range of 0.1-10 %(w/w), such as in the range of 0.5-8 % (w/w), e.g. in the range 1-6 %(w/w), such as in the range of 3-5 %(w/w), e.g. in the range 6-10 %(w/w), such as in the range of 8-10 %(w/w) and/or Ethylhexyl Methoxycinnamate is used in concentrations of at the most 10 %(w/w), e.g. in the range of 0.1-10 %(w/w), such as in the range of 0.5-8 %(w/w), e.g. in the range 1-6 %(w/w), such as in the range of 3-5 %(w/w), e.g. in the range 6-10 %(w/w), such as in the range of 8-10 %(w/w) and/or Octocrylene is used in concentrations of at the most 10 %(w/w), e.g. in the range of 0.1-10 %(w/w), such as in the range of 0.5-8 %(w/w), e.g. in the range 1-6 %(w/w), such as in the range of 3-5 %(w/w), e.g. in the range 6-10 %(w/w), such as in the range of 8-10 % (w/w) and/or 4-tert-Butyl-4'-methoxydibenzoylmethane (BMBM) is used in concentrations of at the most 5 %(w/w), e.g. at the most 2 %(w/w), such as at the most 1%(w/w), e.g. in the range of 0.1-5 %(w/w), such as in the range of 0.1-2 % (w/w), e.g. in the range 0.1-1.5 %(w/w), such as in the range of 0.1-1 %(w/w), e.g. in the range 0.1-0.5 %(w/w), such as in the range of 0.5-5 %(w/w), e.g. in the range 1-5 %(w/w), such as in the range of 2-4 %(w/w), e.g. in the range 2-3 % (w/w), such as in the range of 3-4 %(w/w), such as in the range of 4-5 %(w/w).

Additional sunscreen agents that may be used in connection with the emulsion-type product of the invention include the sunscreen agents that are currently approved for use within the European Community.

The protection which products containing sunscreen agents provide against sunburn is neither absolute nor permanent. One of the many factors that can have an effect on the level of protection given by these products is water contact. UV absorbers in the formulation can leach out or be physically removed by the washing action in the sea or swimming pool

Considerable efforts have therefore been put into making the sunscreen products more effective including the development of formulations which are more substantive to the skin during water immersion. These products have been variously labelled as water resistant and very water resistant. Generally, and in the context of the present invention, a sunscreen product is said to be water resistant if 50% of the static sun protection factor remains after immersion in water two times 20 minutes according to the protocol described herein. Similarly, a sunscreen product is defined as very water resistant if 50% of the static sun protection factor remains after immersion in water four times 20 minutes according to the protocol described herein.

Prior art formulations used as sunscreen products are at best very water resistant in the sense that their static sun protection factor decrease after immersion into water but still remains above 50% of the initial value. However, it is a characteristic of the emulsion-type product according to preferred embodiments of the present invention that, when in the form of a sunscreen product, it has a static sun protection factor after the sunscreen product has been applied to the skin of a human, where the sun protection factor of the sunscreen product is capable of being increased to a higher (wet) sun protection factor when the sunscreen product is contacted with water.

In equally preferred embodiments of the invention the emulsion-type product, once it is applied to a surface, such as the skin of a human, has a non-saturating content of water. Without being bound by theory it is contemplated that the emulsion-type product according to preferred embodiments of the present invention is capable of absorbing additional water after having been applied to the skin. It is further contemplated that the absorption of additional water leads to a thickening of the layer or film formed by the product and, consequently to a significant increase in the distance that the UV radiant must travel through the sunscreen containing film before reaching the skin. Said thickening of the film has no negative impact on the skin feel and the majority of the UV rays are simply converted to heat before reaching the skin.

Consistent with this theory preferred embodiments of the invention provide an emulsion-type product which, once it is applied to a surface, has a content of water which is up to 10% (w/w) less than the saturating content, such as up to 5% (w/w) less than the saturating content, e.g. up to 3% (w/w) less than the saturating content, such as up to 1% (w/w) less than the saturating content, e.g. up to 0.5% (w/w) less than the saturating content. When the surface is the skin of a human the skin-feel of the product is significantly improved and when the product has been applied to the skin, allowed to dry (preferably for about 15 minutes) and subsequently subjected to water it may feels like the product is not even on the skin, Thus, the product does not feel either greasy or sticky.

According to these embodiments it is further preferred that the emulsion-type product once applied to a surface, has a capacity for absorbing up to 10% (w/w) water, such as up to 5% (w/w) water, e.g. up to 3% (w/w) water, such as up to 1% (w/w) water, e.g. up to 0.5% (w/w) water.

Consistent with the US Food and Drug Administration labelling designation for sunscreen products mentioned hereinbefore the sunscreen product of the invention may be designated as a Minimal sun protection product, providing a sun protection factor (SPF) value of 2 to under 12, such as from 4 to 10, from 6 to 10, from 10 to 12, from 4 to 8 or from 8 to 10.

Alternatively the sunscreen product may be a moderate sun protection product, with an SPF value of 12 to under 30, such as from 15-25, from 20-30, from 10 to 20 or from 25-30.

The sunscreen product may also be a high sun protection product, with an SPF value of 30 or above, such as from 30 to 50+, from 30 to 50, from 30 to 40, from 40-50, or form 50-50+ . As it will be apparent to the skilled person, the exact characteristics of the emulsion-type product in this regard depends, *inter alia,* on the specific sunscreen agents that are incorporated into the product.

According to the COLIPA guidelines the sunscreen product of the present invention may be designated as a "Low", "Medium", "High", "Very High" or "Ultra" type product. The COLIPA guidelines for product labelling are presented in Example 4 of the present application. The sunscreen product of the invention may thus have an SPF value of from 2 to 50+, such as an SPF value of 2, 4, 6, 8, 10, 12, 15, 20, 25, 30, 40, 50 or 50+.

Finally, according to a specific embodiment the emulsion-type product according to the invention is a cosmetic product.

Sunscreen product

A sunscreen product is provided which has a static sun protection factor after the sunscreen product has been applied to the skin of a human, where the sun protection factor of the sunscreen product is capable of being increased to a higher (wet) sun protection factor when the sunscreen product is contacted with water. Consistent with the theory explained above the sun protection factor of the product is increased to a higher (wet) sun protection factor subsequent to contact with any source of water, including swimming in fresh water, salt water, showering and sweating. The increase in sun protection factor further occurs irrespective of whether the surface to which the product has been applied is subsequently allowed to dry, preferably by air drying.

In preferred embodiments the higher (wet) sun protection factor of the sunscreen product is increased by at least 5% relative to the static sun protection factor, such as increased by at least 10% relative to the static sun protection factor, e.g. increased by at least 15% relative to the static sun protection factor, such as increased by at least 20% relative to the static sun protection factor, e.g. increased by at least 25% relative to the static sun protection factor, such as increased by at least 30% relative to the static sun protection factor, e.g. increased by at least 35% relative to the static sun protection factor, such as increased by at least 40% relative to the static sun protection factor, e.g. increased by at least 50% relative to the static sun protection factor.

The wet sun protection factor of the sunscreen product is increased by at least 5% relative to the static sun protection factor, such as increased by at least 10% relative to the static sun protection factor, e.g. increased by at least 15% relative to the static sun protection factor, such as increased by at least 20% relative to the static sun protection factor, e.g. increased by at least 25% relative to the static sun protection factor, such as increased by at least 30% relative to the static sun protection factor, e.g. increased by at least 35% relative to the static sun protection factor, such as increased by at least 40% relative to the static sun protection factor, e.g. increased by at least 50% relative to the static sun protection factor when subjected to water for 10 minutes, such as 20 minutes, e.g. 30 minutes, such as 40 minutes, e.g. 50 minutes, such as 60 minutes, e.g. 70 minutes, such as 80 minutes, e.g. 90 minutes, such as 100 minutes.

The sunscreen product is allowed to dry after the sunscreen product has been applied to the skin of a human before being contacted with water. In preferred embodiments, the sunscreen product is allowed to dry for 1 minute or more, such as for 2, 3, 4, 5, 6, 7, 8, 9 or 10 minutes or more. In equally preferred embodiments of the invention the product is allowed to dry to allow evaporation of 70% or more of the mono alcohol Initially present in the product, such as to allow evaporation of 75% or more, 80% or more, 85% or more, 90% or more, 95% or more, or such as to allow evaporation of 98% or more of the mono alcohol initially present in the product.

The sunscreen product comprises at least 10%(w/w) of a mono alcohol having 2-8 carbon atoms. In accordance with the above description of preferred embodiments it may further be preferred that the mono alcohol content of the emulsion type product of the invention is in the range of 10-90%(w/w), such as in the range of 15-90%(w/w), e.g. in the range of 20-85%(w/w), such as in the range of 30-80%(w/w), e.g. in the range of 40-75%(w/w), such as in the range of 50-70%(w/w), e.g. in the range of 55-65%(w/w), such as about 60 %(w/w).

The sunscreen product according to the invention comprises at the most 6%(w/w) water. Further preferred emulsion-type products of the invention may comprise a water content of less than 6%(w/w), such as less than 5%(w/w), such as less than 5%(w/w), such as less than 3%(w/w), such as less than 2%(w/w).

Also preferred is a sunscreen product, wherein the product comprising and at least one water immiscible component. In accordance with the description above the sunscreen product may comprise more than one water immiscible component, such as at least two water immiscible components, e.g. at least 3 water immiscible components, 4 water immiscible components, such at least 5 water immiscible componentls. In certain embodiments of the invention at least one of the water immiscible components present in the product is characterised in it is not miscible with at least one other water immiscible components present in the product. In other words, the product comprises, according to some embodiments of the invention, at least two water immiscible components that are not intermixable.

Further preferred is a sunscreen product as described above, wherein the mono alcohol is an alcohol having 2-4 carbon atoms. In further preferred embodiments the mono alcohol of the sunscreen product has 2-7 carbon atoms, such as 2-6, 2-5, 2-4 or 2-3 carbon atoms. Specifically, it may be preferred that the mono alcohol has 2, 3, 4, 5, 6, 7 or 8 carbon atoms.

According to a presently preferred embodiment of the invention the alcohol in said sunscreen product is an alcohol having an un-branched carbon chain.

In further preferred embodiments of the invention the alcohol is selected from the group consisting of ethanol, 1-propanol, 2-propanol, ethylene glycol, 1-butanol, 2-butanol or any combination thereof.

In other preferred embodiments of the invention the alcohol content of said sunscreen product is in the range of 10-90%(w/w), such as in the range of 15-90%(w/w), e.g. in the range of 20-85%(w/w), such as in the range of 30-80% (w/w), e.g. in the range of 40-75%(w/w), such as in the range of 50-70%(w/w), e.g. in the range of 55-65%(w/w), such as about 60 %(w/w).

In equally preferred embodiments of the invention, the water content of said sunscreen product is in the range of 1-6%(w/w), 2-6%(w/w), 2-5%(w/w), 3-6%(w/w), 3-5%(w/w), 4-6%(w/w), or in the range of 4-5%(w/w).

According to further embodiments of the invention the sunscreen product has a conductivity less than 4 ms/cm, such as a conductivity of 3 ms/cm or less, e.g. a conductivity of 2 ms/cm or less, such as a conductivity of 1 ms/cm or less, e.g. a conductivity of 500 $\mu$s/cm or less, such as a conductivity of 250 $\mu$s/cm or less, e.g. a conductivity of 100 $\mu$s/cm or less, such as a conductivity of 50 $\mu$s/cm or less, when measuring on a mixture of 50%(w/w) product and 50% (w/w) water at 20°C.

In similarly preferred embodiments of the invention the sunscreen product has a pH of 4 or above, e.g. a pH of 5 or above, such as pH 6 or above, e.g. pH 7 or above, such as pH 8 or above, when measuring on a mixture of 50% (w/w) product and 50% (w/w) water at 20°C.

Accordingly, it may be preferred that the product has a pH in the range of 4-8, such as in the range of 5-8, such as in the range of 6-8, such as In the range of 5-8, such as In the range of 5-8, such as in the range of 5-7, such as in the range of 6-9, such as in the range of 6-8, such as in the range of 6-7, e.g. in the range of 7-9 or in the range of 7-8, when measuring on a mixture of 50% (w/w) product and 50% (w/w) water at 20°C.

The sunscreen product according to the invention may further comprise an alkoxylated alcohol. Suitable examples of alkoxylated alcohols are PPG-15 Stearyl ether, PPG-15 and PPG-11 Stearyl ether. In preferred embodiments of the invention the alkoxylated alcohol is PPG-15 Stearyl ether or PPG-15 or a combination thereof.

As for the amount of alkoxylated alcohols present in the sunscreen product according to the invention it may be preferred that the alkoxylated alcohol is added in an amount in the range of 0.1-20%(w/w), such as in the range of 0.5-15%(w/w), e.g. in the range of 1-10%(w/w), such as in the range of 2-7%(w/w), e.g. about 5%(w/w).

In further preferred embodiments according to the invention the sunscreen product further comprises a higher-aliphatic alcohol having 10 carbon atoms or more, Examples of suitable higher-aliphatic alcohols are hexyldecanol and hexyldecyloctadecanol

According to other preferred embodiments of the invention, the higher-aliphatic alcohol having 10 carbon atoms or more is added in an amount in the range of 0.1-20%(w/w), such as in the range of 0.5-15%(w/w), e.g. in the range of 1-10%(w/w), such as in the range of 2-7%(w/w), e.g. about 6%(w/w).

As mentioned hereinbefore, the sunscreen product may further comprise a siloxane or a silane. Suitable examples of silanes and siloxanes are cyclomethicone, cyclohexasiloxane cycloheptasiloxane cyclotrisiloxane and cyclotetrasiloxane. In currently preferred embodiments of the invention the siloxane is cyclic dimethyl polysiloxane or cyclopentasiloxane.

According to the invention, it is preferred that the siloxane or the silane is added in an amount in the range of 0.1-20%(w/w), such as in the range of 0.5-15%(w/w), e.g. in the range of 1-10%(w/w), such as in the range of 2-7% (w/w), e.g. in the range of 2-5%(w/w), such as in the range of 2.5-5%(w/w). Preferably, the siloxane or the silane is added in an amount above 2.5 %(w/w), e.g. above 3 %(w/w), such as above 4 %(w/w), e.g. above 5 %(w/w), such as above 7 %(w/w), e.g. above 8 %(w/w). Alternatively, the siloxane or the silane is added in an amount below 20 %(w/w), e.g. below 15 %(w/w), such as below 10 %(w/w).

According to a currently much preferred embodiments of the invention, the emulsion-type product of the invention comprises a combination of an alkoxylated alcohol, an higher-aliphatic alcohol having 10 carbon atoms or more and/or a siloxane or a silane.

According to the invention it is preferred that the synthetic polymer is added in an amount in the range of 0.1-20%(w/w), such as in the range of 0.5-15%(w/w), e.g. in the range of 1-10%(w/w), such as in the range of 1.5-5%(w/w), e.g. about 2%(w/w).

In further embodiments of the invention the sunscreen product further comprises a hydrophilic colloid (hydrophilic colloid and derivatives and gums). In this context suitable hydrophilic colloids may be selected from the group consisting of: hydroxypropyl ether and hydroxypropylcellulose. In particularly preferred embodiments the hydrophilic colloid is Hydroxypropylcellulose.

According to the invention, the hydrophilic colloid is preferably added in an amount in the range of 0.01-5% (w/w), such as in the range of 0.1-2%(w/w), e.g. in the range of 0.2-1%(w/w), such as in the range of 0.2-0.7%(w/w), e.g. about 0.4%(w/w).

In still further embodiments of the invention the sunscreen product comprises a synthetic polymer. Suitable synthetic polymers may be selected from the group consisting of: acrylate, octylacrylamide and one or more monomers consisting of acrylic acid, methacrylic acid and one of their esters, or combinations thereof. In even more preferred embodiments the synthetic polymer is an acrylate and/or an octylacrylamide copolymer.

According to the invention it is preferred that the synthetic polymer is added in an amount in the range of 0.1-20%(w/w), such as in the range of 0.5-15%(w/w), e.g. in the range of 1-10%(w/w), such as in the range of 1.5-5% (w/w), e.g. about 2%(w/w).

Further preferred embodiments relate to a sunscreen product which further comprises an ester, a benzophenone, a ketone and/or an ether. In currently most preferred embodiments of the invention the ester, the benzophenone, the ketone and/or the ether is selected from the group consisting of: ethylhexyl methoxycinnamate, diethylamino hydroxybenzoyl hexyl benzoate, benzophenone-3, BMBM and octocrylene and any combination thereof

In additionally preferred embodiments of the invention the ester is added to said emulsion-type product in an amount in the range of 0.1-20%(w/w), such as in the range of 0.5-15%(w/w), e.g. in the range of 1-10%(w/w), such as in the range of 2-7%(w/w), e.g. about 5%(w/w).

According to preferred embodiments the sunscreen product, once it is applied to a surface, such as the skin of a human, has a non-saturating content of water.

The sunscreen product once applied to a surface such as the skin of a human, has a capacity for absorbing up to 10% (w/w) water, such as up to 5% (w/w) water, e.g. up to 3% (w/w) water, such as up to 1% (w/w) water, e.g. up to 0.5% (w/w) water.

The sunscreen product may provide sufficient photo protection of the skin of an individual while applied only once a day. In particular, the individual having applied the sunscreen product to the skin once during the morning hours may subsequently remain protected against UV-A and/or UV-B rays during the time spent in the sun on the particular day. According to particular embodiments of the invention, the individual having applied the sunscreen product early during the day may be able to stay in the sun for as long as persistent protection with an SPF value of 20 may allow. In practice, the amount of time during which the individual may sojourn in the sun will be at least 3 hours, such as at least 4, at least 5 at least 6, at least 7, at least 8, at least 9, at least 10, at least 11 or at least 12 hours. As the skilled person will understand the exact amount of time will depend on skin type and climate. Preferably, the protection will persist even though the individual engages in physical activities that involve contact with water, such as swimming, bathing and showering once or multiple times during the day.

The UV-A and/or UV-B filters present are very resistant against UV irradiation. The content of one or more of the UV-A and/or UV-B filter substances are reduced by 50% or less, such as by 45% or less, such as by 40% or less, such as by 35% or less, such as by 30% or less, such as by 25% or less, such as by 20% or less, e.g. by 15% or less, such as by 12% or less, such as by 10% or less, such as by 8% or less, or such as by 5% or less after the product has been irradiated for two hours with a xenon lamp and at a dose of 3 MED/two hours and under the conditions specified in example 4 of the present application.

The sun screen product may have a sun protection factor (SPF) is reduced by 40% or less, such as 35% or less, e.g. 30% or less, such as 28% or less or by 25% or less when the SPF is determined 10 hour after the product has been to the skin of an individual. In this context, the SPF values are determined as recommended by COLIPA and as described in Example 4.

The sunscreen product may have a static sun protection factor (SPF$_{static}$) of 10 or more, such as of 11 or more, e.g. 12 or more, 13 or more, 14 or more, 15 or more, 16 or more, 17 or more, 18 or more, 19 or more, 20 or more, 21 or more, 22 or more, 23 or more, 24 or more, 25 or more or 30 or more. It is further preferred that the specified static sun protection factor may be observed 1 hour or more after application of the sunscreen product, such as 2 hours, 3 hours, 4 hours, 5 hours, 6 hours, 7 hours, 8 hours, 9 hours, 10 hours, 11 hours or 12 hours or more after application of the sunscreen product.

The sunscreen product may have wet sun protection factor (SPF$_{wet}$) of 10 or more, such as of 11 or more, e.g. 12 or more, 13 or more, 14 or more, 15 or more, 16 or more, 17 or more, 18 or more, 19 or more, 20 or more, 21 or more, 22 or more, 23 or more, 24 or more or 25 or more. It is further preferred that the specified wet sun protection factor may be observed 1 hour or more after application of the sunscreen product, such as 2 hours, 3 hours, 4 hours, 5 hours, 6 hours, 7 hours, 8 hours, 9 hours, 10 hours, 11 hours or 12 hours or more after application of the sunscreen product.

Equally preferred are sunscreen products according to the invention which has a percentage water resistance retention of 70% or more, such as of 75% or more, e.g. of 80% or more, 85% or more, 90% or more, 95% or more, 98.5% or more, 100% or more, 102,5% or more, 105% or more, 107,5% or more, 110% or more, 112,5% or more, 115% or more, 117,5% or more, 120% or more, 122,5% or more, 125% or more, 127.5% or more, 130% or more, 132.5% or more, 135% or more, or 137.5% or more, such as a percentage water resistance retention of 140% or more.

Additionally, it is preferred that the specified wet sun protection factor and percentage water resistance may be observed after contact with water at least once, such as at least twice, e.g. at least 3 times, at least 4 times, at least 5 times, at least 6 times, at least 7 times, at least 8 times, at least 9 times or such as at least 10 times. It is to be understood that the length of each interval during which the skin to which the sunscreen product has been applied, may be from 1-10 minutes, from 1-15 minutes, such as from 1-20, 1-30, 1-40 or from 1-60 minutes.

Method for producing an emulsion-type product

A third aspect of the invention provides a method for producing a stable emulsion-type product comprising at least 10%(w/w) of a mono alcohol having 2-8 carbon atoms, 1-6% (w/w) water, a hydrophilic colloid and at least one water immiscible component. The method comprises the steps of:

(i) Providing a water immiscible phase comprising one or more water immiscible components

(ii) mixing the at least 10%(w/w) mono alcohol having 2-8 carbon atoms with the 1-6%(w/w) water, a hydrophilic colloid providing a water/alcohol-mixture, and

(iii) mixing the water immiscible phaseoil mixture from step (I) with the water/alcohol-mixture in step (ii) and obtaining the stable emulsion-type product.

In many embodiments the water immiscible phase comprises more than one water immiscible component. If this is the case, it is preferred to include an additional step of mixing the components of step (i), preferably by stirring.

In preferred embodiments of the invention it is preferred that all of steps (i), (ii) and (iii) as specified above, are performed under stirring. For each step the time and speed of stirring may be optimised as required by the exact conditions, such as the temperature and the desired amount and volume of the product and in consideration of the particular equipment used. It may be preferred that, for each step, the time of stirring may is at least 30 seconds, such as at least 45 seconds, at least 1 minute, at least 1.5 minute, at least 2 minutes, at least 3 minutes, at least 4 minutes, at least 5 minutes, at least 6 minutes, at least 7 minutes, at least 8 minutes, at least 9 minutes, at least 10 minutes, at least 15 minutes, at least 20, minutes, at least 30 minutes, at least 1 hour, at least 2 hours, at least 3 hours, such as at least 4 hours.

In an embodiment of the present invention the time of stirring in step (i) may be performed for at least 20 minutes, such as at least for 40 minutes, e.g. for at least 60 minutes, e.g. for at least 90 minutes, such as in the range of 10 to 90 minutes, e.g. in the range of 30-80 minutes, such as in the range of 45 to 70 minutes, e.g. about 60 minutes.

In an embodiment of the present invention the time of stirring in step (ii) may be performed for at least 30 minutes, such as at least for 60 minutes, e.g. for at least 2 hours, such as for at least 3 hours, e.g. for at least 4 hours, e.g. for at least 5 hours, such as in the range of 30 minutes to 5 hours, e.g. in the range of 2-5 hours, such as in the range of 2 to 4 hours, e.g. in the range of 3-5 hours, such as in the range of 4 to 5 hours, e.g. about 5 minutes.

In an embodiment of the present invention the time of stirring in step (iii) may be performed for at least 30 minutes, such as at least for 60 minutes, e.g. for at least 2 hours, such as for at least 3 hours, e.g. for at least 4 hours, such as in the range of 30 minutes to 4 hours, e.g. in the range of 2-4 hours, such as in the range of 2 to 3 hours, e.g. in the range of 3-4 hours, such as in the range of 1 to 4 hours, e.g. about 2 minutes.

The speed of stirring is preferably from 100-3000 10000 rotations per minute (rpm), such as from 200-9000 rpm, e.g. from 225-8050 rpm, such as from 250-8000 rpm.

In more preferred embodiments it is preferred that the time of stirring in step i) is from 1-20 minutes. In similarly preferred embodiments the speed of stirring in step i) is from 1000-3000 rpm . Further, during step ii) it is preferred that the time of stirring is from 2-20 minutes. It is similarly preferred that the speed of stirring in step ii) is from 2000-10000 rpm) rpm. Finally, in step iii) it is preferred that the time of stirring is 30 minutes and that the speed of stirring is 3000 rpm.

Further preferred embodiments of the invention pertain to a method wherein at least two water immiscible components are mixed in step (i), such as at least 3 water immiscible components, e.g. at least 4 water immiscible components, such as at least 5 water immiscible components, such as at least 6 water immiscible components.

In yet further preferred embodiments the water immiscible components are mixed separately into the mixture.

Consistent with the above description of the emulsion-like product of the invention, certain embodiments provide a method, wherein at least two of the water immiscible components mixed are mutually un-soluble, such as at least 3 of the water immiscible components are mutually un-soluble, e.g. 4 of the water immiscible components are mutually un-soluble, such as at least 5 of the water immiscible components are mutually un-soluble, e.g. 6 of the water immiscible components are mutually un-soluble.

Further embodiments provide a method according to the invention, wherein at least one ester is added, said ester, benzophenone, ketone, and/or ether being selected from the group consisting of a cinnamate, such as ethylhexyl methoxycinnamate, a benzoate, such as diethylamino hydrobenzoyl hexyl benzoate, a crylene, such as octocrylene, benzophenone, such as benzophenone-3 and a ketone, such as BMBM.

Additional embodiments provide a method, wherein the water/alcohol-mixture of step (ii) is added to the oil mixture of step (i).

Further embodiments provide a method, wherein an acrylate copolymer, such as octylacrylamide copolymer, is added to the water immiscible phase obtained in step (i) before the addition of the water/alcohol-mixture in step (iii).

Still further embodiments provide a method wherein a stearyl ether, such as PPG-15 staeryl ether, PPG15 or a combination thereof, is added to the water immiscible phase obtained in step (i) before the addition of the water/alcohol-mixture in step (iii).

In other embodiments relating to the described method hexyldecanol, is added to the water immiscible phase obtained in step (i) before the addition of the water/alcohol-mixture in step (iii).

Also, a polysiloxane, such as cyclic dimethyl polysiloxane or cyclopentasiloxane, may preferably be added to the water immiscible phase obtained in step (i) before the addition of the water/alcohol-mixture in step (iii).

Finally, some currently preferred embodiments of the invention provide a method , wherein a cellulose compound, such as hydroxypropylcellulose, is added to the at least 10%(w/w) mono alcohol having 2-8 carbon atoms in step (ii) before the addition of the 1-6% (w/w) water.

Non-therapeutic use of an emulsion-type product

In relation to this aspect the reader is referred to the above descriptions of preferred embodiments in relation to a sunscreen product.

In particularly preferred embodiments, however, the sunscreen product comprises at least two sunscreen agents, such as 3 sunscreen agents, e.g. 4 sunscreen agents, such as 5 sunscreen agents, e.g. 6 sunscreen agents.

Further, it will be understood that the at least one sunscreen agent reduces UVA-rays and UVB-rays. It may further be preferred to combine, in the emulsion-type product of the invention, one or more sunscreen agents which are capable of reducing UV-A rays with one or more sunscreen agents which are capable of reducing UV-B rays.

It should be noted that, according to the present invention, embodiments and features described in the context of one of the aspects of the present invention also apply to the other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Additionally, although individual features may be included in different claims, these may possibly be advantageously combined, and the inclusion in different claims does not imply that a combination of features is not feasible and/or advantageous. In addition, singular references do not exclude a plurality. Thus, references to "a", "an", "first", "second etc. do not preclude a plurality. Furthermore, reference signs in the claims shall not be construed as limiting the scope.

The following figures and examples are included to demonstrate particular embodiments of the invention.

Figure 1: shows the transmission and absorption of UV light as a function of the sun protection factor (SPF).

Figure 2: shows the sun protection factor (SPF) as a function of waiting time.

**EXAMPLES**

Example 1: Preparation of an emulsion-type product comprising UVA and UVB filters.

Ingredients

| Steps | Ingredients | amount w/w % |
|---|---|---|
| 2.1 | Alcohol denat. (solvent) | 56-58 |
| 2.,3 | Aqua (Solvent) | 4.0-5.0 |
| 2.2 | Hydroxypropylcellolose (Emulsion stabilizer/film former/thickening agent, a hydrophilic colloid) | 0.3-0.5 |
| 1.4 | Acrylates/Octylacrylamide Copolymer (Film former, a synthetic polymer) | 1.8-2.2 |
| 1.2 | PPG-15 Stearyl Ether (Emolient/weak emulsifier, an alkoxylated alcohol) | 4.5-5.5 |

(continued)

| Steps | Ingredients | amount w/w % |
|---|---|---|
| 1.3 | Hexyldecanol (Emollent, an higher-aliphatic alcohol) | 5-7 |
| 1.1 | Cyclopentasiloxane (Emolient, a Siloxane/silane) | 4.5-5.5 |
| 1.5 | Ethylhexyl Methoxycinnamate (Sunscreen agent, an ester) | 4.7-5.0 |
| 1.6 | Ethylhexyl Methoxycinnamate (and) Diethylamino Hydroxybenzoyl Hexyl Benzoate (Sun screen agent, a sun screen agent/ester + amine/ester/phenol) | 3.5-4.5 |
| 1.7 | Octocrylene (Sun screen agent, an ester) | ≤10 |
| | Total | 100 |
| | SPF | 21 |
| | WRR% (water resistance retention) | 71 |

Method of preparation:

Step 1.

| | Equipment: Laboratory- and pilot planet dissolver Dispermat AE04-C1, 80 mm propeller | Period of stirring | rpm |
|---|---|---|---|
| 1. | Addition of Cyclopentasiloxane to mixing vessel, initiate stirring | | |
| 2. | PPG-15 Stearyl ether is added under stirring conditions | 1 min. | |
| 3. | Hexyldecanol is added under stirring conditions | 1 min. | 230 |
| 4. | Acrylates/Octylacrylamide Copolymer is added slowly under stirring conditions | 4 min. | 300 |
| 5. | Ethylhexyl Methoxycinnamate is added under stirring conditions | 3 min. | 300 |
| 6. | Ethythexyl Methoxycinnamate (and) Diethylamino Hydroxybenzoyl Hexyl Benzoate is added under stirring conditions | 3 min. | 350 |
| 7. | Octocrylene is added under stirring conditions | 10 min. | 450 |

Step 2.

| | Equipment: Silverson L4RT | Period of stirring | rpm |
|---|---|---|---|
| 1 | Addition of Alcohol Denat. to mixing vessel | | |
| 2. | Addition of Hydroxypropylcellolose | 2 min. | 8100 |
| 3. | Addition of Aqua | 4 min. | 8100 |

Step 3.

| | Equipment Laboratory- and pilot planet dissolver Dispermat AE04-C1, 80 mm propeller | Period of stirring | Rpm |
|---|---|---|---|
| 1. | Mixture from step 1 was transferred to a clean vessel and stirring was initiated | | |

(continued)

| | Equipment Laboratory- and pilot planet dissolver Dispermat AE04-C1, 80 mm propeller | Period of stirring | Rpm |
|---|---|---|---|
| 2. | Mixture from step 2 was added slowly under stirring conditions. | 7 min. | 700 |

Example 2:

Determination of the sun protection factor for an emulsion-type product comprising UVA and UVB filters as provided in example 1.

The principle of the water resistance test is to compare the Sun Protection Factor for a sunscreen product after a period of immersion in water with the original, static SPF of the product determined according to the International Sun Protection Factor (SPF) Test Method. The Sun Protection Factor value on an individual subject ($SPF_I$), for any product, either before or after water immersion, is defined as the ratio of the minimum erythemal dose on protected skin ($MED_P$) to the minimum erythemal dose on unprotected skin ($MED_U$) of the same subject.

The static SPF ($SPF_s$) was determined according to the current published document entitled "International Sun Protection Factor (SPF) Test Method" from 2003. All references to "The International Sun Protection Factor (SPF) Test Method" herein, relate to that document.

To determine the wet SPF ($SPF_w$), the International Sun Protection Factor (SPF) Test Method was followed to the point where the product under test has been applied to the volunteer's skin. Product treated skin is then immersed in water according to the following schedule:

- Allow 15 to 30 minutes drying time after product application.
- First 20 minute immersion in water
- Allow 15 minutes drying time (No towelling)
- Second 20 minute immersion in water
- Allow to dry for 15 minutes or until completely dry (No towelling)

The method for determining water resistance utilized a spa-pool. Use of a spa-pool in the water immersion procedure is preferred over the use of a swimming pool as the latter renders it difficult to standardize the procedure. The equipment which was used complied with the guidelines describing the procedure for water immersion. The SPF measurement procedure was that described by the International Sun Protection Factor.

On completion of the water immersion procedure, the International Sun Protection Factor (SPF) Test Method is resumed at the point immediately after the product was applied and allowed to dry on the volunteer's skin. This then completes the wet SPF determination. This wet SPF can then be compared with the SPF obtained for product treated skin, not immersed in water (the static SPF).

Test Subject Selection

Members of the volunteer panel were selected to fulfill the selection criteria with regard to skin type, skin condition, medical condition and time since last test, as laid out in the International Sun Protection Factor (SPF) Test Method. For each test subject there was a minimum rest period of two months before participation in a new water resistance retention test or any other test involving exposure of the skin to ultraviolet radiation (eg SPF test).

Test Area

The anatomical test area was restricted to the back, between the scapula line and the waist, with the exact locations chosen to ensure complete submersion in the immersion equipment. Test product application to test sites was randomized on each individual subject and over the whole test panel.

Source of Ultraviolet (UV) Radiation

An artificial source of UV radiation (solar simulator) was used, conforming to the specification for an appropriate artificial UV source as defined in the International Sun Protection Factor (SPF) Test Method. Control of the UV source quality was according to the COLIPA Guidelines for Monitoring UV-Light Sources.

Reference Water Resistant Sun Product

The procedures as described in this example was validated by testing the Standard Water Resistant Sun Product coded P2. A fresh sample of P2, not older than 12 months of age, was used. The expected level of water resistance was > 50%. According to the guidelines validation is required at least every four months.

The formula, manufacturing instructions, stability and physiochemical specifications for the water resistant reference product (P2) were as follows: (P2)

Formula Details of P2:

| Ingredients | INCI Name | % W/W |
|---|---|---|
| Phase 1 | Lanolin | 4.5 |
|  | Theobroma Cacao | 2.0 |
|  | Glyceryl Stearate ("Glyceryl Monostearate SE") | 3.0 |
|  | Stearic Acid | 2.0 |
|  | Octyl Dimethyl PABA | 7.0 |
|  | Benzophenone-3 ("Oxybenzone") | 3.0 |
| Phase 2 | Aqua (water) | 71.6 |
|  | Sorbitol | 5.0 |
|  | Triethanolamine | 1.0 |
|  | Methylparaben | 0.3 |
|  | Propylparaben | 0.1 |
| Phase 3 | Benzyl Alcohol | 0.5 |

Manufacturing Process

i.     Melt the ingredients of the fatty Phase 1 and heat to 80-85°C.
ii.    Heat Phase 2 to 80-85 °C, until completely solubilised.
iii.   Add Phase 1 into Phase 2, while stirring Phase 2 with a homogeniser (Moritz type).
iv.    Cool to 50°C while stirring, then add Benzyl Alcohol and complete cooling. Compensate for water loss and homogenize

Physiochemical Specifications

| Appearance: | White yellowish fluid emulsion. |
|---|---|
| pH: | 8.6±0.5 |
| Viscosity: | 2.5 poises (at 10mn, Contraves NB rheometer, rotary body N°3) |
| Density: | 0.95 g/ml |

Analytical Data

HPLC:   Octyl Dimethyl PABA    6.9 to 7.1 % w/w

(continued)

| Benzophenone-3 | 2.8 to 3.2 % w/w |
|---|---|

Photometric Data

Typical data for a 100 mg/l solution in Isopropanol:

| | | | |
|---|---|---|---|
| λmax.: | 309.4 nm | Abs. Max.: | 0.909 |
| λ: | 290.0 nm | Abs.: | 0.540 |
| λ: | 320.0 nm | Abs.: | 0.671 |
| λ: | 340.0 nm | Abs.: | 0.120 |
| λ: | 400.0 nm | Abs.: | 0.000 |

Formulation was stable for least 2 months at 45°C and 12 months at 20°C.

Expected SPF and Water Resistance Values of the formulation are: SPF 12 to 15, Water Resistance > 50%

Product Quantity and Application

The emulsion-type product as described in Example 1 was applied at a precise application rate of 2.0 mg/cm$^2$ ($\pm$2.5%). The amount of product applied and the uniformity of spreading on the test sites is known to affect the magnitude and variability of the test results. The recommendations set out in the International Sun Protection Factor (SPF) Test Method for product application quantity, product application technique and minimal area of the product application site were therefore followed closely.

Determination of the Minimum Erythemal Dose (MED)

Minimum erythemal doses (MEDs) were determined according to the appropriate section of the current International Sun Protection Factor (SPF) Test Method. The dose increment used to determine the unprotected and protected MEDs for the wet SPF were the same as used to determine the MEDs for the static SPF.

SPF measurements before and after water immersion ('static' and 'wet' SPF) were determined in the same laboratory on the same panel of volunteers as part of the same test sequence. For each test of the emulsion-type product 12 to 16 volunteers were selected and all subjects completed the test.

Water Immersion Procedure

The procedure for water immersion required that the entire product treated site was fully submersed in water for various defined periods of time. The current studies employed a spa-pool, which together with a Jacuzzi or bathtub with water circulation is the only recommended equipment.

Equipment Specific to procedure (immersion equipment):

Spa-pool with sufficient capacity to enable complete immersion of all test areas with no contact between subject and any surface.
Means of controlling water temperature.
Ease of cleaning, sanitizing, filling and emptying should be ensured.
Security: Non-slip coatings were used and volunteers were supervised at all times.

Water Quality

The immersion equipment was filled with water complying with EC Council Directive 98/83/EC (Yd November 1998) governing the quality of water intended for human consumption. The levels of magnesium and calcium were below the maximum permitted level for magnesium and calcium (combined) of 500mg/l and above the minimum level of 50 mg/l. The temperature of the water was 29°C $\pm$ 2°C.

The water in the immersion equipment was sanitised with bromide or chlorine according to the manufacturer's recommendations. Alternatively, where no chemical sanitation procedure is adopted, the equipment was emptied, cleaned and refilled between each volunteer that used the pool. The spa-pool was emptied and refilled every day during testing activity.

Immersion Conditions

Test product was applied to the test area (back) designated for water immersion according to the current International Sun Protection Factor (SPF) Test Method. 15 to 30 minutes drying time was allowed to elapse after application of the test product to the skin and before immersion in water.

The immersion equipment was furnished with a means of continuous water circulation which does not direct water onto the test areas and produces a rate of flow which adequately simulates moderate activity.

The volunteer was then immersed in the immersion equipment for 20 minutes ensuring complete immersion of the test sites under the water and avoiding contact between any test site and any part of the immersion equipment.

The volunteer exited the immersion equipment after the first 20-minute immersion and the product treated site was allowed to air-dry (with no towelling) for a further period of 15 minutes.

The volunteer was then immersed in the immersion equipment for a second period of 20 minutes, again ensuring complete immersion of the test sites under the water and that there is no contact between any test site and any part of the immersion equipment.

The volunteer exited the immersion equipment after the second 20-minute immersion and the product treated site was allowed to air-dry (with no towelling) for a further period of 15 minutes or until completely dry. It was determined that there were no visible water drops on the test site at the end of this drying period.

After the skin had completely dried, the SPF of the product applied to the test sites and immersed in water was then determined according to the International Sun Protection Factor (SPF) Test Method.

Further volunteers were recruited and the same procedure followed until sufficient volunteers have generated sufficient data to comply with the statistical criteria.

Test procedure and chronology

The sequence in which the static and wet SPFs are determined may be critical and therefore the static and wet SPFs were determined in the sequence described in the following:

Test Chronology and Procedure

The following procedure was followed for each volunteer and was repeated on between 10 and up to 20 volunteers, until statistical criteria were satisfied.

• Day 1

(i)  Unprotected, static MED ($MED_S$) was determined on previously unexposed skin.

(ii)  Product was applied to designated static SPF sites ($MED_P$, [Product(a)); $MED_P$, [Product(b)] ...etc.).

(iii)  15 to 30 minutes was allowed for the product to dry.

(iv)  The product treated static sites ($MED_P$, [Product(a)), $MED_P$, [Product(b)) ...etc.) were exposed to progressive UV doses according to the International Sun Protection Factor (SPF) Test Method.

• Day 2

(i)  The erythemal reactions from previous day's UV exposures on unprotected static $MED_{us}$ sites were assessed and the static $MED_{us}$ for unprotected, non-immersed skin was determined.

(ii)  The erythemal reactions from previous day's UV exposures on product protected static $MED_{ps}$ sites were assessed and the static $MED_{PS}$ for product protected, non-water-immersed skin was determined.

(iii)  The individual static SPF ($SPF_{is}$) was calculated.

(iv)  Product was applied to test sites designated for water immersion ($MED_{pw}$ [Product(a)], $MED_{pw}$ [Product (b)] ...etc.).

(v)  Product was allowed to dry for 15 to 30 minutes.

(vi)  Volunteer was Immersed in water for 20 minutes with continuous water circulation.

(vii)  Volunteer was removed from immersion equipment and test sites were allowed to air-dry with no toweling for 15 minutes.

(continued)

(viii)   Volunteer was returned to immersion equipment and immersed in water for a further 20 minutes with continuous water circulation.

(ix)   Volunteer was removed from immersion equipment and test sites were allowed to air-dry with no toweling for 15 minutes or until test site was completely dry. It wais ensured that all test sites were free from water droplets. If water droplets were present, air-drying time was extended.

(x)   Product treated water immersion sites ($MED_{pw}$ [Product(a)], $MED_{pw}$ ]Product(b)] ...etc.) were exposed to progressive UV doses according to the International Sun Protection Factor (SPF) Test Method.

(xi)   Unprotected MED ($MED_{uw}$) was repeated. This unprotected MED is post water-immersion, so as to be compatible with the protected wet $MED_{pw}$.

• Day 3

(i)   The erythemal reactions from previous day's UV exposures on unprotected wet $MED_{uw}$ sites and determine $MED_{uw}$ for unprotected skin, were assessed after water immersion.

(ii)   The erythemal reactions from previous day's UV exposures on product protected, wet $MED_{pw}$ sites were assessed and $MED_{PW}$ for product protected, water-immersed skin was determined.

(iii)   Individual wet SPF ($SPF_{pw}$) was calculated according to the International Sun Protection Factor (SPF) Test Method.

Calculation and data handling

The following calculations were performed:

*Mean static SPF (SPFs)*

The static SPF ($SPF_S$) was calculated as the mean of the total individual static SPF values ($SPF_{is}$), determined on all subjects completing the procedure. A corresponding 95% bilateral confidence interval (95%CI) was also calculated. Both the SPFs and the 95%CI were calculated according to the guidelines described in the International Sun Protection Factor (SPF) Test Method. A test was considered acceptable if the 95% confidence interval on the mean static SPF ($SPF_s$) was within $\pm$ 17% of the mean static SPF ($SPF_s$).

For further details of statistical definitions, reference is made to the International Sun Protection Factor (SPF) Test Method.

*Individual Percentage Water Resistance Retention*

An individual Percentage Water Resistance Retention (%WRR1) value was calculated for each individual subject according to the formula below:

$$\%WRR_I = ((SPF_{iw}-1)/(SPF_{is}-1)) \times 100$$

Where:

SPF$_{iw}$ = individual wet SPF after water immersion
SPF$_{is}$ = individual static SPF

*Mean Percentage Water Resistance Retention*

The mean percentage water resistance retention (%WRR) was expressed as the arithmetic mean of the 'n' individual %WRR values (%WRR;).

*Calculation of Lower Confidence Limit on the Mean Percentage Water Resistance Retention*

The confidence in the value for the mean percentage water resistance retention (%WRR) was expressed by way of a unilateral 90% confidence interval. The unilateral confidence interval was used because it was only necessary to predict the lower confidence level for the mean %WRR, since this is all that was needed to be confident that the product performs to a minimum level of water resistance. An upper confidence limit was not required, because it was not important to know the upper level of water resistance performance in this test. The level of confidence was set as 90% rather than the conventional 95% to allow for the increased biological variability that arose from the two major steps in the measurement procedure. In addition to the variability arising from the SPF determination (which is usually assigned a 95% confidence) there was the additional variability that arose from the water immersion procedure. In recognition of this increased, expected variability, a 90% confidence level was chosen rather than 95%.

The 90% unilateral confidence interval for the mean %WRR was calculated as:

$$[\text{mean\%WRR-d}]$$

with d calculated as:

$$d = \frac{t_u \times s}{\sqrt{n}}$$

where

s = standard deviation

n = total number of volunteers in test

$t_u$ = t value from the 'one-sided' Student-t distribution table at a probability level

p=0.10 and with n-1 degrees of freedom

| n | 10 | 11 | 12 | 13 | 14 | 15 | 16 | 17 | 18 | 19 | 20 |
|---|---|---|---|---|---|---|---|---|---|---|---|
| $t_u$ values | 1.383 | 1.372 | 1.363 | 1.356 | 1.350 | 1.345 | 1.341 | 1.337 | 1.333 | 1.330 | 1.328 |

*Acceptance Criteria for water resistance testing*

A product is considered water resistant if the value for the 90% lower unilateral confidence limit [mean %WRR - d] is greater than or equal to 50% AND the 95% confidence interval on the mean static SPF was within $\pm$ 17% of the mean static SPF.

*Very Water Resistant Claims*

In addition to being tested according to the process for water resistant products as outlined above, products which are designed to provide extra water resistance should also be tested by adding a further two twenty-minute water immersion periods to the water immersion procedure described above, in the paragraph "Immersion Conditions". The revised water immersion procedure for very water resistance testing is then:

- Allow 15 to 30 minutes drying time after product application.
- First 20 minute immersion in water
- Allow 15 minutes drying time (No towelling)
- Second 20 minute immersion in water
- Allow 15 minutes drying time (No towelling)
- Third 20 minute immersion in water
- Allow 15 minutes drying time (No towelling)
- Fourth 20 minute immersion in water
- Allow to dry for 15 minutes or until completely dry (No towelling)

After completion of the water immersion procedure, the SPF test is resumed as for the standard water resistance test and the calculations described in section 4 are performed. A product will be considered very water resistant if the value for the 90% lower unilateral confidence limit [mean %WRR - d] is greater than or equal to 50% AND the 95% confidence interval on the mean static SPF was within $\pm$ 17% of the mean static SPF.

Results

International - SPF - Test Method 2003/Sun Product Water Resistance 2004
Ctfa - COLIPA - JCIA
Water Immersion 4 x 20 minutes
Data of analysis
Institut Dr. Schrader Hautphysiologie, April 7th, 2006

Table 1: Shows the information on the individual volunteers and the effect of the reference product P2.

| Characterization of Subjects | | | | | | European Standard P2 | | Data Rejection Criterion |
|---|---|---|---|---|---|---|---|---|
| No | Initials | Age | Sex | Type | ITA° | MEDp ($mJ/cm^2$) | SPF | |
| 1 | S.S. | 22 | f | I | 57 | 354.0 | 17.0 | |
| 2 | J.O. | 51 | f | II | 52 | 376.9 | 21.3 | |
| 3 | S,B | 21 | f | I | 58 | 247.5 | 15.2 | No $MED_P$ value |
| 4 | E.K. | 64 | f | II | 50 | 311.2 | 17.0 | No $MED_P$ value |
| 5 | E.P. | 44 | f | II | 43 | 303.4 | 13.6 | |
| 6 | C.P. | 44 | f | II | 42 | 307.4 | 12.1 | No $MED_P$ value |
| 7 | U.R. | 49 | f | II | 44 | 336.0 | 15.2 | |
| 8 | M.M. | 44 | f | II | 48 | 253.4 | 12.1 | No $MED_P$ value |
| 9 | N.S. | 31 | f | II | 54 | 370.3 | 19.0 | |
| 10 | C.C. | 45 | f | II | 46 | 365.6 | 17.0 | |
| 11 | L,D. | 40 | f | II | 50 | 346.3 | 17.0 | |
| 12 | M,G. | 45 | f | II | 45 | 370.6 | 21.3 | |
| 13 | K.C. | 35 | f | II | 48 | 284.9 | 12.1 | |
| 14 | L.A. | 25 | f | II | 47 | 267.2 | 10.8 | |
| 15 | A.B. | 38 | f | II | 44 | 297.5 | 12.1 | |
| 16 | P.H. | 35 | f | II | 52 | 379.2 | 17.0 | |
| Number | | | | | | 16 | 16 | |
| Mean | | | | | | 322.6 | 15.6 | |
| Standard Deviation | | | | | | 46.0 | 3.3 | |
| Confidence Interval (CI) - Deviation from Mean | | | | | | | 1.8 | |
| Confidence Interval (CI) - Deviation from Mean [%] | | | | | | | 11.3 | |
| Lower Limit of CI | | | | | | | 13.8 | |
| Upper Limit of CI | | | | | | | 17.4 | |
| Label SPF/WRR | | | | | | | | |

Table 2: shows the static and the wet Sun Protection Factor (SPF) based on the same 16 individual volunteers as in table 1 and the effect of the product according to the present invention and as provided in example 1. The wet Sun Protection Factor (SPF$_w$) was evaluated after water immersion for 4x20 minutes.

| Subject No. | Static | | | Wet (Spa Pool) | | | Water resistance [%] |
|---|---|---|---|---|---|---|---|
| | MEDu (mJ/cm) | MEDp (mJ/cm$^2$) | SPF$_{static}$ | MEDu (mJ/um) | MEDp (mJ/cm) | SPF$_{wet}$ | |
| 1 | 20.8 | 371.8 | 17.9 | 20.8 | 419.8 | 20.2 | 113.7 |
| 2 | 17.7 | 443.4 | 25.1 | 17.7 | 447.0 | 25.3 | 100.8 |
| 3 | 16.3 | 260.0 | 15.9 | 14.6 | -- | > 25 | > 163 |
| 4 | 16.3 | 326.9 | 17.9 | 18.3 | -- | > 25 | > 144 |
| 5 | 22.4 | 447.7 | 20.0 | 22.4 | 451.3 | 20.2 | 100.8 |
| 6 | 25.4 | 508.0 | 20.0 | 25.4 | -- | > 20 | > 101 |
| 7 | 22.1 | 442.7 | 20.0 | 22.1 | 499.8 | 22.6 | 113.6 |
| 8 | 20.9 | 373.9 | 17.9 | 20.9 | -- | > 23 | > 128 |
| 9 | 19.5 | 435.7 | 22.4 | 19.5 | 435.7 | 22.4 | 100.0 |
| 10 | 21.5 | 384.0 | 17.9 | 21.5 | 481.7 | 22.4 | 126.9 |
| 11 | 20.4 | 363.7 | 17.9 | 20.4 | 511.0 | 25.1 | 142.9 |
| 12 | 17.4 | 488.4 | 28.1 | 15.5 | 547.0 | 35.2 | 128.4 |
| 13 | 23.5 | 527.3 | 22.4 | 21.0 | 527.3 | 25.1 | 112.6 |
| 14 | 23.8 | 476.2 | 20.0 | 18.9 | 476.2 | 28.1 | 142.6 |
| 15 | 24.6 | 491.7 | 20.0 | 22.0 | 605.8 | 27.6 | 140.0 |
| 16 | 22.3 | 398.3 | 17,9 | 19.9 | 490.7 | 24.6 | 140.2 |
| Number | 16 | 16 | 16 | 16 | 12 | 12 | 12 |
| Mean | 21.1 | 421.2 | 20.1 | 19.9 | 491.1 | 24.9 | 121.7 |
| Standard Deviation | 2.7 | 72.1 | 3.1 | 2.8 | 52.3 | 4.1 | 17.0 |
| Confidence Interval (CI) - Deviation from Mean | | | 1.7** | | | 2.6 ** | 6.7 * |
| Confidence Interval (CI) - Deviation from Mean [%] | | | 8.3 | | | 10.5 | 5.5 |
| Lower Limit of CM | | | 18.4 | | | 22.2 | 115.0 |
| Upper Limit of CI | | | 21.8 | | | 27.6 | 128.4 |

(continued)

| Subject No. | Static | | | Wet (Spa Pool) | | | Water resistance [%] |
|---|---|---|---|---|---|---|---|
| | MEDu (mJ/cm) | MEDp (mJ/cm$^2$) | SPF$_{static}$ | MEDu (mJ/um) | MEDp (mJ/cm) | SPF$_{wet}$ | |
| Label SPF/WRR | | | 20 | | | | 115 |
| WRR - Water Resistance Retention; * 90% one sided; ** 95% two sided | | | | | | | |

Conclusion

The above experiment demonstrates that the Sun Protection Factor of the product according to the present invention may be increased when subjected to water (see table 2 - SPF$_{static}$ and SPF$_{wet}$).

Furthermore, the experiment demonstrates that the sun screen product according to the present invention and as prepared in example 1 has a high water resistance retention whereby it is sufficient to apply the sun screen product only one time a day, providing a "Once-A-Day" product.

Example 3:

Determination of the pH, conductivity and appearance for the product of the present invention. Different dilutions of the emulsion-type product with water and alcohol are provided.

The following dilutions of the emulsion-type product with water and alcohol (ethanol) were prepared in 100 ml Bluecap tubes. The dilutions were mixed by manual shaking.

All percentage values are W/W%

All weighted amounts are within $\pm$ 0.01 g from the desired amount.

Table 3: illustrates the pH, conductivity and appearance for the product of the present invention. The product tested had the basic composition as described in Example 1 and was further diluted with varying contents of water.

| Amount water added | Density | Temperature when measuring density | pH | Temperature when measuring pH | Conductivity | Appearance |
|---|---|---|---|---|---|---|
| 0% | 0.8645 g/ml | 23.9°C | 6.95 | 23.6°C | 18.7 $\mu$S/cm | Clear yellow liquid |
| 5% | 0.8747 g/ml | 24.2°C | 6.55 | 25.1°C | 25.9 $\mu$S/cm | Clear yellow liquid |
| 6% | 0.8768 g/ml | 24.5°C | 6.68 | 24.6°C | 26.9 $\mu$S/cm | Yellow liquid, cloudy appearance |
| 7% | 0.8786 g/ml | 24.75°C | 6.69 | 24.7°C | 28.3 $\mu$S/cm | Yellow liquid, cloudy appearance |
| 10% | 0.8840 g/ml | 24.70°C | 6.67 | 24.4°C | 31.4 $\mu$S/cm | Precipitate is formed |
| 20% | 0.8967 g/ml | 27.2°C | 7.18 | 27.0°C | 43.7 $\mu$S/cm | Precipitates, white/light yellow appearance |

(continued)

| Amount water added | Density | Temperature when measuring density | pH | Temperature when measuring pH | Conductivity | Appearance |
|---|---|---|---|---|---|---|
| 30% | 0.9109 g/ml | 27.2°C | 7.22 | 27.1°C | 48.6 µS/cm | Precipitates, white/light yellow appearance |
| 40% | 0.8989 g/ml | 27.4°C | 7.18 | 27.1°C | 46.3 µS/cm | Precipitates, white/light yellow appearance |
| 50% | 0.9440 g/ml | 27.8°C | 7.40 | 27.4°C | 49.3 µS/cm | Precipitates, white/light yellow appearance |
| 20% | 0.8954 g/ml | 25.8°C | 6.96 | 26.3°C | 43.9 µS/cm | Precipitates, white/light yellow appearance |
| 30% | 0.9081 g/ml | 25.9°C | 7.09 | 27.0°C | 45.5 µS/cm | Precipitates, white/light yellow appearance |
| 40% | 0.9307 g/ml | 25.6°C | 7.27 | 26.9°C | 46.6 µS/cm | Precipitates, white/light yellow appearance |
| 50% | 0.9453 g/ml | 25.7°C | 7.38 | 26.4°C | 47.3 µS/cm | Precipitates, white/light yellow appearance |

Table 4: illustrates the pH, conductivity and appearance for the product of the present invention. The product tested had the basic composition as described in Example 1 and was further diluted with varying contents of Alcohol Denaturant.

| Amount of ethanol added | Density | Temperature when measuring density | pH | Temperature when measuring pH | Conductivity | Appearance |
|---|---|---|---|---|---|---|
| 0% | 0,8645 g/ml | 23.9°C | 6.95 | 23.6°C | 18.7 µS/cm | Clear yellow liquid |
| 5% | 0.8613 g/ml | 24.7°C | 6.78 | 25.6°C | 18.2 µS/cm | Clear yellow liquid |
| 6% | 0.8602 g/ml | 24.8°C | 6.86 | 24.4°C | 18.3 µS/cm | Clear yellow liquid |
| 7% | 0.8597 g/ml | 25.0°C | 6.87 | 22.8°C | 18.4 µS/cm | Clear yellow liquid |

(continued)

| Amount of ethanol added | Density | Temperature when measuring density | pH | Temperature when measuring pH | Conductivity | Appearance |
|---|---|---|---|---|---|---|
| 10% | 0.8577 g/ml | 25.0°C | 6.88 | 23.4°C | 18.5 μS/cm | Clear yellow liquid |
| 20% | 0.8494 g/ml | 25.1°C | 6.91 | 24.1°C | 17.9 μS/cm | Clear yellow liquid |
| 30% | 0.8413 g/ml | 25.1 °C | 6.99 | 24.4°C | 17.5 μS/cm | Clear yellow liquid |
| 40% | 0.8337 g/ml | 25.1°C | 7.03 | 24.7°C | 16.7 μS/cm | Clear yellow liquid |
| 50% | 0,8255 g/ml | 25.2°C | 7.11 | 24.2°C | 15.5 μS/cm | Clear yellow liquid |

The data illustrate that the emulsion-type product can hold up towards 6% water while still being completely clear. At a content of 6% water the product starts to appear cloudy but is still stable. When reaching a content of 10% water precipitates begin to form. Furthermore, the data illustrates that the conductivity of the product is low, namely, less than 50 μS/cm and that the pH-value of the product is rather constant around pH 7 ($\pm 0.5$).

Example 4: Determination of photo stability

Sample Preparation

The test sample are weighed and distributed evenly and homogeneously with a brush onto the marked area of a glass plate.

Setting up the Irradiation Lamp

For the Irradiation we use a Xenon arc lamp because the continuous spectrum in the UV-region is near-identical to that of-natural light. The lamp is turned on approximately one hour before Irradiation.

Irradiation

The glass plate Is positioned under the lamp so that the square with the test sample is maximally illuminated. [0201] The distance (lamp - glass plate) is acquired with the aid of a calibrated hand radiometer (PMA 2100) with UV-B detector. The radiant Intensity of the lamp on the sample is set up so that an Irradiation dose of 3 MED (Minimal Erythemal Dose) is given after two hours.

Determining the Concentration of the UV-Filter Substances

In order to ascertain to which extent each of the Individual UV-filters in the tested formulations has been photometrically degraded, they are analytically quantified before and after irradiation.

The instrumental-analytical method used is HPLC

Sample is prepared as described in Example 1

Results:

| Determined UV-Filter Substances | Content of UV-Filter Substances [%] | | | |
| --- | --- | --- | --- | --- |
| | Before irradiation | | After irradiation [3MED/2h] | |
| | Declared | Found [%] | Found [%] | [%] rel. |
| Octocrylene | 10,0 | 9,98 | 9,35 | 93,7 |
| Ethylhexyl Methoxycinnamate | 4,6+ (4-x) | 7,61 | 4,65 | 61,1 |
| Diethylamino Hydroxybenzoyl Hexyl Benzoate | x | 1,29 | 1,21 | 93,8 |

Discussion

In both formulations is observed a relatively strong degradation of the UV-B filter Ethylhexyl Methoxy¬cinnamate. This is confirmed through a lot of other experiments which show the same results with a stronger or strong degradation of cinnamic compounds. However, it should be noted that there is normally a strong UV mediated degradation of cinnamic compounds and when the degradation of this compound is tested in sunscreen formulations only a relative amount of approximately 30% will be expected to remain after irradiation as specified. The other UV-B filter (Octocrylene) and the UV-A filter (Diethylamino Hydroxybenzoyl Hexyl Benzoate) show under the specific experimental conditions very good value of photo stability.

Determination of Sun Protection Factor according to the international Sun Protection Factor Test Method.

Summary

Sun protection factors for the test product according to I-SPF-TM, n=12

| Product | Measurement Time | Mean Value | Recommended Claim | |
| --- | --- | --- | --- | --- |
| | | SPF | SPF | Protection |
| 30/6-2005 Bl. 5e | Baseline | 20.1 | 20 | High Protection |
| 30/6-2005 Bl. 5e | 2h | 20.1 | 20 | High Protection |
| 30/6-2005 Bl. 5e | 4 h | 17.4 | 15 | High Protection |
| 30/6-2005 Bl. 5e | 6 h | 17.1 | 15 | High Protection |
| 30/6-2005 Bl. 5e | 8 h | 16.6 | 15 | High Protection |
| 30/6-2005 Bl. 5e | 10 h | 17.4 | 15 | High Protection |
| Test persons: 12 Sex: 9 female, 3 male Age: 18 - 53 years | | | | |

Details of the experiment

Selection of volunteers

The volunteers taking part in this test were selected on the basis of Fitzpatrick's skin type table and on the basis of skin colour typing by colorimetric measurements respectively. They corresponded to the majority of users as far as their skin sensitivity classification is concerned. The following types resulted:

Skin colour typing

| Phototype | Skin colour typing | ITA° value* |
|---|---|---|
| Type I | Very light | >55° |
| Type II | Light | from 42 to 55° |
| Type III | Intermediate | from 29 to 41° |
| Type IV | Tan (or matt) | from 11 to 28° |
| Type V | Brown | - 30 to 10° |
| Type VI | black | < - 30° |
| * ITA° = [Arc Tangent (L'-50/b')]] 180/3.14159 | | |

The test product was tested on a panel, which consisted of nine female and three male volunteers aged between 18 and 53 years.

Determining the sun protection factors

The test was based on the International-SPE-Test Method (I-SPF-TM) as defined in 2003 (2, 3).

Test areas on the volunteers' backs of 6 x 6 cm are coated with $2 \pm 0.05$ mg/cm2 of the test product. The quantity applied is controlled by reweighing. Between product application and irradiation there is a waiting time of 15 minutes. $20 \pm 4$ hours after the end of irradiation the resultant erythema is used to ascertain the minimum erythema dose (MED). The individual sun protection factor is determined from the ratio of MEDu (MED of the untreated skin) and MED of the area treated with the product in question.

Number of volunteers

The number of volunteers is restricted to a minimum of 10 and a maximum of 20 valid results in the International-SPF Test Method. A minimum of 10 volunteers is sufficient If the 95 0/0 confidence Interval Of the mean sun protection factor is within $\pm$ 17 0/0 of the mean. Otherwise, the number of volunteers is increased from 10 until the statistical criterion is met.

A maximum of five individual results may be excluded from the calculation of mean SPF, but each exclusion has to be justified. All individual results are included in this report.

Amount of application

The I-SPF-TM recommends an amount of application of $2.0 \pm 0.05$ mg/cm2. The amounts of application are determined with the aid of a precision balance before and after product application.

Ambient conditions

Product application, UV-exposures and MED assessment were carried out in stable ambient conditions. Testing was performed in an air-conditioned laboratory with a room temperature of $24 \pm 1°C$.

Incremental progression of UV-dose

A geometric progression of 1.25 or 1.12 can be used. If the expected SPF is above 25 a geometric progression of 1.12 has to be used.

Data rejection criteria

Test data are rejected under the following circumstances:

i) The exposure series on a subject fails to elicit an erythemal response on any sub-site, $20 \pm 4$ hours after exposure.
ii) Erythemal responses within an exposure series are randomly absent $20 \pm 4$ hours after exposure.
iii) All sub-sites In the exposure series show an erythemal response $20 \pm 4$ hours after exposure.

Data presentation

In order to take into account the statistical variation of a screening test, results are presented on the basis of the 95% Confidence Interval (950/0-CI).

Kinetic

In a special time regime excitation of test areas is carried out 20 min (Standard), 2 h, 4 11, 6 h, 8 h and 10 h after product application. Volunteers stay in the lab without any covering clothes of the test area. No special activity is allowed during the waiting time of this investigation.

A result obtained from the Intemational-SPF-Test Method

The results obtained using the International SPF-TM method for SPF measurement were expressed as a mean SPF with confidence Interval for 95% probability.

Labelling of SPF Number and Optional Product Categories

Based on the COLIPA Recommendation N°19 (April 2005) labelling of sunscreens should be carried out as follows:

The mean SPF found should be used as basis for labelling. When labelling very high SPF numbers (ie above SPF 25) consideration should be given to the potential for variability in SPF numbers.

The mean value from the test is rounded down to the nearest number in the SPF Classification Table shown below (Table 5), and this number is the maximum SPF to be labelled. An SPF number lower than the nearest rounded down SPF value may be used to label the product but the labelled value should take account of the lower 95 % confidence interval. SPF test results should not be rounded up to the nearest number in the classification table.

The lower SPF limit is 2. Any sun protection should provide a minimum SPF of at least 2.

The maximum SPF labelled should be SPF 50+ (for a product to be labelled as SPF 504-the mean SPF measured must have been SPF 60 or above).

The SPF numbers labelled are restricted to those and only those shown in the table.

The following Categories and SPF numbers are recommended by COLIPA. Indication of products Categories may be useful and should be optional.

Table 5: Categories of possible SPF numbers

| Type | SPF |
|------|-----|
| Low | 2 / 4 / 6 |
| Medium | 8 / 10 / 12 |
| High | 15 / 20 / 25 |
| Very High | 30 / 40 / 50 |
| Ultra | 50+ |

Sun protection factor difference

The principle of sunscreens is based on reducing the transmission of UV radiation into the deeper layers of the skin by means of absorption or reflection. It is there° le essential to take non-linear absorption processes into consideration. In this manner a SPF 5 means a remaining transmission of UV light of 20 %, SPF 10 means 10 %, SPF 20 means 5 % etc. I

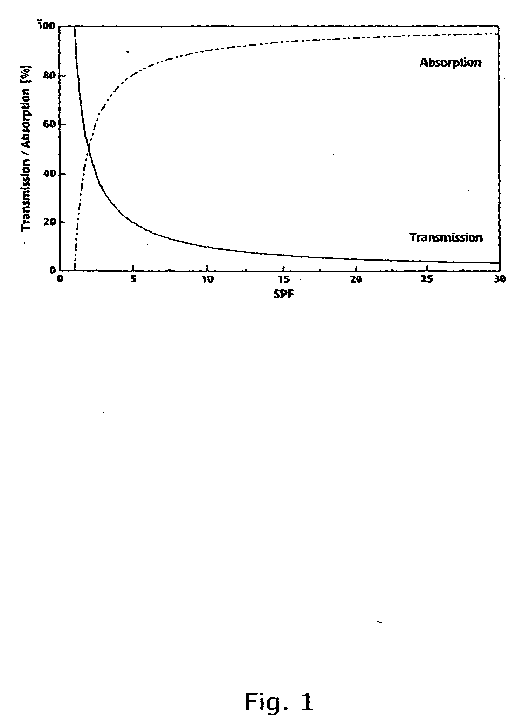

Differences in high sun protection factors (SPF 15) are frequently interpreted in the same way as for lower sun protection factors. The non-linearity of the SPF is not taken into consideration, as Fig. 1 shows. The difference in transmission for sun protection factors which are one unit apart, for instance SPF 5 and SPF 6, is approx. 3.3 0/0_ if this 3.3 0/0 difference in transmission is transferred to higher sun protection factors, then the difference between SPF 5 and SPE 6 is identical to that between SPF 10 and SPF 15, or SPF 15 and SPF 30. The example given above shows that differences in sun protection factors are, because of the reciprocity, relatively dependent on the absolute level of the SPF in question.

Excitation spectrum

A 300 W Xenon light source is used by the Multlport-601 system (Solar Light Company) for excitation. By especial filtering with a WG 320 and a UG 11 filter the accuracy of the International-SPF-Test Method for the excitation source is guaranteed. These criteria are checked frequently. The excitation spectrum is recorded with a PMT detection system (photo-multiplier-tube and double monochromator). From this the erythemal effectiveness of each wavelength band is calculated as a percentage of the total erythema' effectiveness from < 290 to 400 nm (Relative Cumulative Erythemal Effectiveness) and compared to the guidelines. The detection system 15 checked frequently with a standardization source having a known spectrum. This guarantees an accurate Intemational-SPF-Test Method spectrum at any time (4, 5).

Results and discussion

Sun protection factor

An average was formed from the individual sun protection factor for the test product, the details are summarized in the following table.

Table 6: Sun protection factors for the test product 30/6-2005 BI. 5e

| Product | Measurement Time | Mean Value | Recommended Claim | |
|---|---|---|---|---|
| | | SPF | SPF | Protection Category |
| 30/6-2005 BI. 5e | Baseline | 20.1 | 20 | High Protection |
| 30/6-2005 BI. 5e | 2 h | 20.1 | 20 | High Protection |
| 30/6-2005 BI. 5e | 4 h | 17.4 | 15 | High Protection |
| 30/6-2005 BI. 5e | 6 h | 17.1 | 15 | High Protection |
| 30/6-2005 BI. 5e | 8 h | 16,6 | 15 | High Protection |
| 30/6-2005 BI. 5e | 10 h | 17.1 | 15 | High Protection |

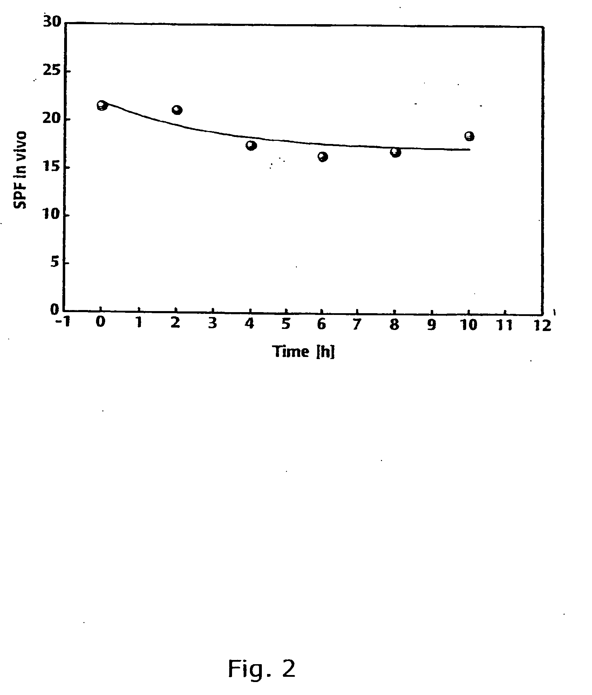

In this investigation the test product achieves a mean SPF of 20.1 with a 95 % confidence interval of 17.7 - 22.5. By this a label SPF of 20 can be recommended.
In this investigation the test product (2 h) achieves a mean SPF of 20.1 with a 95% confidence Interval of 18.1 - 22.0. By this a label SPF of 20 can be recommended.
In this investigation the test product (4 h) achieves a mean SPF of 17.4 with a 95% confidence Interval of 15.5 - 191. By this a label SPF of 15 can be recommended.
In this investigation the test product (6 h) achieves a mean SPF of 17.1 with a 95% confidence Interval of 15.6 - 18.6. By this a label SPF of 15 can be recommended.
In this investigation the test product (8 h) achieves a mean SPF of 16.6 with a 95% confidence interval of 15.0 - 18.2. By this a label SPF of 15 can be recommended.
In this Investigation the test product (10 h) achieves a mean SPF of 17.1 with a 95% confidence interval of 15.5 - 18.6. By this a label SPF of 15 can be recommended.
A graphical presentation of the kinetic SPF-behaviour is given In figure 2.
As can be seen in figure 2 only a slight decay of the SPF is obtained for the test product Based on this kinetic investigation for the product only a slight decrease in SPF from SPF 20 to SPF 17 is measured 10 h after product application.
Example 5: This experiment is provided to demonstrate the effect of water and hydroxypropylcellulose on the stability of the emulsion-type products of the present invention.

Emulsion-type products tested

Test emulsion-type product 1 has the same composition as described in Example 1.
Test emulsion-type product 2 has the same composition as described in Example 1 except that the product does not contain water (content of water is 0 %(w/w)).

Test emulsion-type product 3 has the same composition as described in Example 1 except that the content of water is 12 %(w/w).

Test emulsion-type product 4 has the same composition as described in Example 1 except that the product does not contain hydroxypropylcellulose (content of hydroxypropylcellulose is 0 %(w/w)).

All the Test emulsion-type products are mixed in a one litre glass-bottle and left at room temperature in the laboratory without direct sunlight and with no agitation.

The samples were visually investigated for their appearance.

Results observed

| Mixture | Aberration from the product in Example 1 | Description of appearance instantly after preparation | Description of appearance two weeks after preparation |
|---|---|---|---|
| 1 | Same as Example 1 | Clear and yellow gel; | Clear and homogeneous gel |
| 2 | Water = 0 %(w/w) | Distinctly unclear gel | Unclear, heterogeneously gel: several small gel-particles are floating in the mixture |
| 3 | Water = 12 %(w/w) | Distinctly unclear mixture with fluffy precipitate | Distinctly unclear mixture with a great deal of solid precipitate, which is hard to unsettle |
| 4 | hydroxypropylcellulose = 0% (w/w) | Clear mixture; very thin liquid | Clear mixture; very thin liquid |

Conclusion

As apparent from the above results water and hydroxypropylcellulose need to be present in the present emulsion-type product to provide a clear gel product. If the water content is reduced to 0 %(w/w) the product becomes unclear, unstable as several particles are formed. On the other hand when the water content is increased to 12 %(w/w) the product is also unstable and solid particle even starts to form. If the content of hydroxypropylcellulose is reduced to 0 %(w/w) the emulsion-type product seems to be stable, however, the product becomes a very thin liquid and the sunscreen effect was demonstrated to be reduced significantly from a SPF of above 21 to a SPF of approximately 16, thus, the sun proactive effect is significantly reduced.

**Claims**

1.  A stable emulsion-type product comprising at least 10%(w/w) of a mono alcohol having 2-8 carbon atoms, a water content in the range of 1-6% (w/w), a hydrophilic colloid and at least one water immiscible component.

2.  An emulsion-type product according to any one of the preceding claims, wherein the product has a conductivity less than 4 ms/cm, when measuring on a mixture of 50%(w/w) product and 50%(w/w) water at 20°C, or wherein the product has a pH of 4 or above when measuring on a mixture of 50% (w/w) product and 50% (w/w) water at 20°C.

3.  An emulsion-type product according to any one of the preceding claims, wherein the product further comprises an alkoxylated alcohol, a higher-aliphatic alcohol having 10 carbon atoms or more, and/or a siloxane or a silane.

4.  An emulsion-type product according to any one of the preceding claims, wherein the hydrophilic colloid is hydroxypropylcellulose.

5.  An emulsion-type product according to any one of the preceding claims, wherein the product further comprises a synthetic polymer, an ester, a benzophenone, a ketone and/or an ether.

6.  An emulsion-type product according to any one of the preceding claims, wherein the product is a sunscreen product, a cosmetic product or a carrier for an active ingredient.

7. A method for producing a stable emulsion-type product comprising at least 10%(w/w) of a mono alcohol having 2-8 carbon atoms, a water content in the range of 1-6% (w/w), a hydrophilic colloid and at least one water immiscible component, said method comprises the steps of:

(i) Providing a water immiscible phase comprising one or more water immiscible components
(ii) mixing the at least 10%(w/w) mono alcohol having 2-8 carbon atoms with the at most 6%(w/w) water and the hydrophilic colloid providing a water/alcohol-mixture, and
(iii) mixing the water immiscible phase from step (i) with the water/alcohol-mixture in step (ii) and obtaining the stable emulsion-type product.

8. A method according to claim 7, wherein at least two water immiscible components are mixed in step (i) or wherein the water immiscible components are mixed separately into the mixture.

9. A method according to claims 7 or 8, wherein at least two of the water immiscible components mixed are mutually un-soluble.

10. A method according to any one of claims 7-9, wherein at least one ester, at least one benzophenone, at least one ketone and/or at least one ether is added, said ester, benzophenone, ketone and/or ether being selected from the group consisting of a cinnamate, a benzoate, a crylene, a benzophenone, a ketone, and/or any combination hereof.

11. A method according to any one of claims 7-10, wherein an acrylate copolymer, such as octylacrylamide copolymer, and/or a stearyl ether, such as PPG-15 stearyl ether, PPG15 or a combination thereof, and/or hexyldecanol, and/or a polysiloxane, such as cyclic dimethyl polysiloxane or cyclopentasiloxane, and/or is added to the water immiscible phase obtained in step (i) before the addition of the water/alcohol-mixture in step (iii).

12. A method according to any one of claims 7-11, wherein the cellulose compound, is hydroxypropylcellulose, is added to the at least 10%(w/w) mono alcohol having 2-8 carbon atoms in step (ii) before the addition of the at most 6% (w/w) water.

13. Non-therapeutic use of an emulsion-type product according to claims 1-12, as a carrier substance for an active ingredient.

**Patentansprüche**

1. Stabiles Produkt vom Emulsionstyp, umfassend mindestens 10 Gew.-% eines einwertigen Alkohols mit 2-8 Kohlenstoffatomen, einen Wassergehalt im Bereich von 1-6 Gew.-%, ein hydrophiles Kolloid und mindestens einen nicht mit Wasser mischbaren Bestandteil.

2. Produkt vom Emulsionstyp nach einem der vorhergehenden Ansprüche, wobei das Produkt beim Messen einer Mischung aus 50 Gew.-% Produkt und 50 Gew.-% Wasser bei 20 °C eine Leitfähigkeit von weniger als 4 ms/cm aufweist oder wobei das Produkt beim Messen einer Mischung aus 50 Gew.-% Produkt und 50 Gew.-% Wasser bei 20 °C einen pH-Wert von 4 oder mehr aufweist.

3. Produkt vom Emulsionstyp nach einem der vorhergehenden Ansprüche, wobei das Produkt weiterhin einen alkoxylierten Alkohol, einen höheraliphatischen Alkohol mit 10 Kohlenstoffatomen oder mehr und/oder ein Siloxan oder ein Silan umfasst.

4. Produkt vom Emulsionstyp nach einem der vorhergehenden Ansprüche, wobei das hydrophile Kolloid Hydroxypropylcellulose ist.

5. Produkt vom Emulsionstyp nach einem der vorhergehenden Ansprüche, wobei das Produkt weiterhin ein synthetisches Polymer, einen Ester, ein Benzophenon, ein Keton und/oder einen Ether umfasst.

6. Produkt vom Emulsionstyp nach einem der vorhergehenden Ansprüche, wobei das Produkt ein Sonnenschutzprodukt, ein kosmetisches Produkt oder ein Träger für einen Wirkstoff ist.

7. Verfahren zur Herstellung eines stabilen Produkts vom Emulsionstyp, umfassend mindestens 10 Gew.-% eines einwertigen Alkohols mit 2-8 Kohlenstoffatomen, einen Wassergehalt im Bereich von 1-6 Gew.-%, ein hydrophiles Kolloid und mindestens einen nicht mit Wasser mischbaren Bestandteil, wobei das Verfahren folgende Schritte umfasst:

(i) Bereitstellen einer nicht mit Wasser mischbaren Phase, umfassend einen oder mehrere nicht mit Wasser mischbare Bestandteile

(ii) Mischen der mindestens 10 Gew.-% des einwertigen Alkohols mit 2-8 Kohlenstoffatomen mit den höchstens 6 Gew.-% Wasser und dem hydrophilen Kolloid zur Bereitstellung einer Wasser-Alkohol-Mischung

(iii) Mischen der nicht mit Wasser mischbaren Phase aus Schritt (i) mit der Wasser-Alkohol-Mischung aus Schritt (ii) und Erhalten des stabilen Produkts vom Emulsionstyp.

8. Verfahren nach Anspruch 7, wobei in Schritt (i) mindestens zwei nicht mit Wasser mischbare Bestandteile gemischt werden oder wobei die nicht mit Wasser mischbaren Bestandteile getrennt in die Mischung eingemischt werden.

9. Verfahren nach Anspruch 7 oder 8, wobei mindestens zwei der eingemischten nicht mit Wasser mischbaren Bestandteile wechselseitig nicht ineinander löslich sind.

10. Verfahren nach einem der Ansprüche 7-9, wobei mindestens ein Ester, mindestens ein Benzophenon, mindestens ein Keton und/oder mindestens ein Ether zugegeben wird, wobei der Ester, das Benzophenon, das Keton und/oder der Ether ausgewählt ist aus der Gruppe, bestehend aus einem Cinnamat, einem Benzoat, einem Crylen, einem Benzophenon, einem Keton und/oder einer Kombination davon.

11. Verfahren nach einem der Ansprüche 7-10, wobei der in Schritt (i) erhaltenen nicht mit Wasser mischbaren Phase vor der Zugabe der Wasser-Alkohol-Mischung in Schritt (iii) ein Acrylat-Copolymer, wie Octylacrylamid-Copolymer, und/oder ein Stearylether, wie PPG-15 Stearylether, PPG15 oder eine Kombination davon, und/oder Hexyldecanol und/oder ein Polysiloxan, wie cyclisches Dimethylpolysiloxan oder Cyclopentasiloxan, zugegeben wird.

12. Verfahren nach einem der Ansprüche 7-11, wobei die Celluloseverbindung Hydroxypropylcellulose den mindestens 10 Gew.-% des einwertigen Alkohols mit 2-8 Kohlenstoffatomen vor der Zugabe der höchstens 6 Gew.-% Wasser zugegeben wird.

13. Nicht therapeutische Verwendung eines Produkts vom Emulsionstyp nach den Ansprüchen 1-12 als Trägersubstanz für einen Wirkstoff.

**Revendications**

1. Produit de type émulsion stable comprenant au moins 10 % (p/p) d'un monoalcool possédant 2 à 8 atomes de carbone, une teneur en eau située dans la plage de 1 à 6 % (p/p), un colloïde hydrophile et au moins un composant non miscible avec l'eau.

2. Produit de type émulsion selon l'une quelconque des revendications précédentes, dans lequel le produit possède une conductivité inférieure à 4 ms/cm, mesurée sur un mélange de 50 % (p/p) de produit et 50 % (p/p) d'eau à 20 °C, ou dans lequel le produit possède un pH supérieur ou égal à 4 mesuré sur un mélange de 50 % (p/p) de produit et 50 % (p/p) d'eau à 20 °C.

3. Produit de type émulsion selon l'une quelconque des revendications précédentes, dans lequel le produit comprend également un alcool alcoxylé, un alcool aliphatique supérieur possédant au moins 10 atomes de carbone et/ou un siloxane ou un silane.

4. Produit de type émulsion selon l'une quelconque des revendications précédentes, dans lequel le colloïde hydrophile est de l'hydroxypropylcellulose.

5. Produit de type émulsion selon l'une quelconque des revendications précédentes, dans lequel le produit comprend également un polymère synthétique, un ester, une benzophénone, une cétone et/ou un éther.

6. Produit de type émulsion selon l'une quelconque des revendications précédentes, dans lequel le produit est un produit écran solaire, un produit cosmétique ou un support pour un ingrédient actif.

7. Méthode de production d'un produit de type émulsion stable comprenant au moins 10 % (p/p) d'un monoalcool possédant 2 à 8 atomes de carbone, une teneur en eau située dans la plage de 1 à 6 % (p/p), un colloïde hydrophile et au moins un composant non miscible avec l'eau, ladite méthode comprenant les étapes suivantes :

(i) apport d'une phase non miscible avec l'eau comprenant au moins un composant non miscible avec l'eau,
(ii) mélange des au moins 10 % (p/p) d'un monoalcool possédant 2 à 8 atomes de carbone avec les au plus 6 % (p/p) d'eau et le colloïde hydrophile pour obtenir un mélange eau/alcool, et
(iii) mélange de la phase non miscible avec l'eau de l'étape (i) avec le mélange eau/alcool de l'étape (ii) et obtention du produit de type émulsion stable.

8. Méthode selon la revendication 7, dans laquelle au moins deux composants non miscibles avec l'eau sont mélangés à l'étape (i) ou dans laquelle les composants non miscibles avec l'eau sont mélangés séparément avec le mélange.

9. Méthode selon les revendications 7 ou 8, dans laquelle au moins deux des composants non miscibles avec l'eau mélangés sont insolubles l'un dans l'autre.

10. Méthode selon l'une quelconque des revendications 7 à 9, dans laquelle au moins un ester, au moins une benzophénone, au moins une cétone et/ou au moins un éther est ajouté, ledit ester, benzophénone, cétone et/ou éther étant choisi dans le groupe composé d'un cinnamate, d'un benzoate, d'un crylène, d'une benzophénone, d'une cétone et/ou de toute combinaison de ceux-ci.

11. Méthode selon l'une quelconque des revendications 7 à 10, dans laquelle un copolymère d'acrylate, tel qu'un copolymère d'octylacrylamide, et/ou un éther stéarylique, tel qu'un éther stéarylique de PPG-15, du PPG15 ou une combinaison de ceux-ci, et/ou de l'hexyldécanol, et/ou un polysiloxane, tel que du diméthylpolysiloxane cyclique ou du cyclopentasiloxane, et/ou est ajouté à la phase non miscible avec l'eau obtenue à l'étape (i) avant l'addition du mélange eau/alcool à l'étape (iii).

12. Méthode selon l'une quelconque des revendications 7 à 11, dans laquelle le composé cellulosique est de l'hydroxypropylcellulose, est ajouté aux au moins 10 % (p/p) d'un monoalcool possédant 2 à 8 atomes de carbone à l'étape (ii) avant l'addition des au plus 6 % (p/p) d'eau.

13. Usage non thérapeutique d'un produit de type émulsion selon les revendications 1 à 12, comme substance de support pour un ingrédient actif.

Fig. 1

Fig. 2